# EUROPEAN PATENT SPECIFICATION

(11) **EP 0 640 601 B1**
(45) Date of publication and mention of the grant of the patent: **04.12.1996**
(21) Application number: 92904979.9
(22) Date of filing: 12.02.1992
(51) Int. Cl.: C07D 451/04, C07D 451/14, C07D 207/09, C07D 211/58, C07D 265/30, A61K 31/40, A61K 31/46, A61K 31/435, A61K 31/535, C07D 207/14, C07D 211/42, C07D 211/26, C07D 279/12

(54) **BENZAMIDE DERIVATIVE**
BENZAMID-DERIVATE
DERIVE DE BENZAMIDE

(30) Priority: 15.02.1991 JP 42425/91; 22.08.1991 JP 233756/91
(43) Date of publication of application: 01.03.1995
(73) Proprietor: HOKURIKU SEIYAKU CO., LTD., Katsuyama-shi, Fukui-ken 911 (JP)
(72) Inventor: ITO, Yasuo, Katsuyama-shi Fukui-ken 911 (JP); KATO, Hideo, Fukui-shi Fukui-ken 910 (JP); YASUDA, Shingo, Katsuyama-shi Fukui-ken 911 (JP); IWASAKI, Nobuhiko, Katsuyama-shi Fukui-ken 911 (JP); NISHINO, Hiroyuki, Katsuyama-shi Fukui-ken 911 (JP); TAKESHITA, Makoto, Katsuyama-shi Fukui-ken 911 (JP)
(74) Representative: Werner, Hans-Karsten, Dr.Dipl.-Chem.
(86) International application number: JP9200134
(87) International publication number: WO9214705

(56) References cited:
- EP-A- 0 099 194
- EP-A- 0 230 718
- EP-A- 0 243 959
- JP-A- 1 050 883
- JP-A-52 122 379
- JP-A-58 010 578
- JP-A-61 063 642

## Description

### Technical Field

The present invention relates to novel benzamide derivatives which have excellent gastrointestinal stimulating activity and antiemetic activity and are useful as medicament for the treatment of gastrointestinal diseases. The present invention further relates to a method for preparing said benzamide derivatives and a pharmaceutical composition useful for the treatment of gastrointestinal diseases comprising said benzamide derivative as an active ingredient.

### Background Art

Since the development of Metoclopramide (The Merck Index, 11th Edition, 6063) as an antiemetic agent and a gastrointestinal prokinetic agent, various substituted benzamide derivatives having antiemetic activity and gastrointestinal stimulating activity have been provided.

For example, The Merck Index (11th edition, 2344) discloses Clebopride which is a benzamide derivative having a piperidine ring and is useful as antiulcer agent. The Merck Index (11th edition, 2318) discloses Cisapride useful as a gastrointestinal prokinetic agent. These drugs are widely used clinically. In addition, Japanese Patent Unexamined Publication No. (Sho)55-92384 discloses that benzamide derivatives substituted with an azabicyclo ring have antiemetic activity and gastrointestinal stimulating activity and that they are useful as an antiemetic agent and a gastrointestinal prokinetic agent.

Document D1 (EP-A-0 243 959) discloses substituted benzamide derivatives such as 4-amino-5-chloro-N-[(4-substituted-2-morpholinyl)methyl]-2-methoxybenzamide, pharmaceutical compositions containing the same, and processes for the preparation thereof. In said compounds, salts and N-oxide derivatives thereof show good gastro-intestinal motility enhancing activity.

Document D2 (EP-A-0 230 718) describes substituted benzamides and benzoates, having pharmacological activity, processes for the preparation thereof and their use as pharmaceuticals.

Document D3 (EP-A-0 099 194) discloses benzamides which have anti-depressant and/or anxiolytic activity, a process for their preparation and their use in the treatment of mammals.

However, these substituted benzamide derivatives are not sufficiently useful since they do not have stimulating activity on lower digestive tracts such as the large intestine, although they have stimulating activity on upper digestive tracts such as the stomach. Furthermore, these substituted benzamide derivatives are not satisfactory from a safe standpoint since they sometimes cause adverse reactions such as extrapyramidal syndrome or hyperprolactinemia.

Accordingly, an object of the present invention is to provide medicament for the treatment of gastrointestinal diseases having stimulating activity not only on upper digestive tract but on lower digestive tract and reduced side effects.

The inventors of the present invention conducted various studies to achieve the aforementioned object, and found that novel benzamide derivatives of the present invention have stimulating activity both on upper digestive tract and lower digestive tract. The inventors also found that these novel benzamide derivatives have reduced side effects and are useful as medicament for the treatment of gastrointestinal diseases having excellent selectivity. The present invention was achieved on the basis of these findings.

### Disclosure of the Invention

The present invention provides benzamide derivatives represented by the following formula (I): 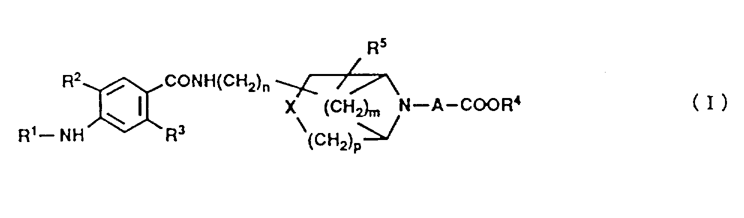 wherein, R¹ represents a hydrogen atom or a C₁-C₄ alkanoyl group; R² represents a hydrogen atom or a halogen atom; R³ represents a C₁-C₄ alkoxy group; R⁴ represents a hydrogen atom or a C₁-C₄ alkyl group; R⁵ represents a hydrogen atom, a C₁-C₄ alkyl group, or a C₁-C₄ alkoxy group; A represents C₁-C₇ alkylene group which may optionally be substituted with a C₁-C₄ alkyl group; X represents a methylene group, an oxygen atom, or a sulfur atom; m represents an integer of from 0 to 3; n represents an integer of from 0 to 3; and p represents an integer of from 0 to 2, or pharmacologically acceptable salts thereof. The benzamide derivatives are useful since they have gastrointestinal stimulating activity.

According to another embodiment of the present invention, a method for preparing said benzamide derivatives is provided.

According to yet another embodiment of the present invention, a pharmaceutical composition useful for the treatment of gastrointestinal diseases which comprises said benzamide derivative as an active ingredient. The pharmaceutical composition is useful as, for example, a gastrointestinal prokinetic agent, an antiemetic agent, an agent for the treatment of irritable bowel syndrome, and agent for the treatment of constipation.

### Best Mode for Carring Out the Invention

In the aforementioned formula (I), a C₁-C₄ alkanoyl group represented by R¹ may be, for example, formyl group, acetyl group, propanoyl group, butyroyl group, or trimethylacetyl group. A halogen atom represented by R² may be, for example, a fluorine atom, a chlorine atom, or a bromine atom. A C₁-C₄ alkoxy group represented by R³ and R⁵ may be, for example, methoxy group, ethoxy group, propoxy group, isopropoxy group, butoxy group, isobutoxy group, or tert-butoxy group. A C₁-C₄ alkyl group represented by R⁴ and R⁵ or a C₁-C₄ alkyl group which may optionally substitute on the alkylene group represented by A may be, for example, methyl group, ethyl group, n-propyl group, isopropyl group, n-butyl group, or tert-butyl group. R⁵ and Ph-CONH(CH₂ ) ₙ - (wherein Ph represents a substituted phenyl) may substitute at any position of a ring comprising X and N.

Examples of pharmacologically acceptable salts of the benzamide drivatives of the present invention include alkali-addition salts or acid-addition salts. Examples of the alkali-addition salts include, for example, inorganic alkali-addition salts such as, for example, sodium salt, potassium salt, calcium salt, and ammonium salt, and organic base-addition salts such as, for example, ethylenediamine salt, ethanolamine salt, N,N-dialkylethanolamine salt, and triethanolamine salt. Examples of the acid addition salts include, for example, inorganic acid-addition salts such as, for example, hydrochloride, hydrobromide, hydroiodide, sulfate, nitrate, and phosphate, and organic acid-addition salts such as, for example, acetate, maleate, fumarate, citrate, oxalate, tartrate, malate, mandelate, methanesulfonate, p-toluenesulfonate, and 10-camphorsulfonate.

The benzamide derivatives of the present invention represented by the above-described formula (I) may optionally have an asymmetric carbon atom. Each of enantio-isomers distinguished by the asymmetric carbon atom, an arbitrary mixture thereof, and racemate, i.e., equimolor mixture thereof, fall within the scope of the present invention. Where the benzamide derivatives of the present invention have more than one asymmetric carbon atoms, each diastereomer and an arbitrary mixture of the diastereomers also fall within the scope of the present invention.

The benzamide derivatives of the present invention may be in any one of possible conformations. For instance, where the compounds of the aforementioned formula (I) wherein p is 1, piperidine, morphorine, or thiamorphorine ring substituted with Ph-CONH(CB₂ ) ₙ - group may be in any one of configurations selected from chair form, boat form, or twist-boat form. In the specification, compounds having Ph-CONH(CH₂ ) ₙ - group and -(CH₂ ) ₘ - cross linking group above the same side of a ring plane comprising X and N are referred to as endo-compounds, and compounds having each of the groups above the opposite sides of the plane are referred to as exo-compounds.

Preferred embodiments of the benzamide derivatives of the present invention include the following compounds:
(1) ethyl exo-[3-(4-acetylamino-2-methoxybenzamido)-8-azabicyclo[3.2.1]oct-8-yl]acetate;
(2) ethyl exo-4-[3-(4-acetylamino-2-methoxybenzamido)-8-azabicyclo[3.2.1]oct-8-yl]butyrate;
(3) ethyl endo-[3-(4-acetylamino-2-methoxybenzamido)-8-azabicyclo[3.2.1]oct-8-yl]acetate;
(4) ethyl endo-4-[3-(4-acetylamino-2-methoxybenzamido)-8-azabicyclo[3.2.1]oct-8-yl]butyrate;
(5) ethyl exo-[3-(4-acetylamino-2-methoxybenzamido)-9-azabicyclo[3.3.1]non-9-yl]acetate;
(6) ethyl exo-4-[3-(4-acetylamino-2-methoxybenzamido)-9-azabicyclo[3.3.1]non-9-yl]butyrate;
(7) ethyl endo-[3-(4-acetylamino-2-methoxybenzamido)-9-azabicyclo[3.3.1]non-9-yl]acetate;
(8) ethyl endo-3-[3-(4-acetylamino-2-methoxybenzamido)-9-azabicyclo[3.3.1]non-9-yl]propionate;
(9) ethyl endo-4-[3-(4-acetylamino-2-methoxybenzamido)-9-azabicyclo[3.3.1]non-9-yl]butyrate;
(10) ethyl endo-5-[3-(4-acetylamino-2-methoxybenzamido)-9-azabicyclo[3.3.1]non-9-yl]valerate;
(11) methyl endo-6-[3-(4-acetylamino-2-methoxybenzamido)-9-azabicyclo[3.3.1]non-9-yl]hexanoate;
(12) methyl endo-7-[3-(4-acetylamino-2-methoxybenzamido)-9-azabicyclo[3.3.1]non-9-yl]heptanoate;
(13) methyl endo-8-[3-(4-acetylamino-2-methoxybenzamido)-9-azabicyclo[3.3.1]non-9-yl]octanoate;
(14) ethyl endo-[3-(4-acetylamino-2-ethoxybenzamido)-9-azabicyclo[3.3.1]non-9-yl]acetate;
(15) ethyl endo-4-[3-(4-acetylamino-2-ethoxybenzamido)-9-azabicyclo[3.3.1]non-9-yl]butyrate;
(16) ethyl exo-[3-(4-acetylamino-5-chloro-2-methoxybenzamido)-8-azabicyclo[3.2.1]oct-8-yl]acetate;
(17) ethyl exo-4-[3-(4-acetylamino-5-chloro-2-methoxybenzamido)-8-azabicyclo[3.2.1]oct-8-yl]butyrate;
(18) ethyl endo-[3-(4-acetylamino-5-chloro-2-methoxybenzamido)-8-azabicyclo[3.2.1]oct-8-yl]acetate;
(19) ethyl endo-4-[3-(4-acetylamino-5-chloro-2-methoxybenzamido)-8-azabicyclo[3.2.1]oct-8-yl]butyrate;
(20) ethyl exo-[3-(4-acetylamino-5-chloro-2-methoxybenzamido)-9-azabicyclo[3.3.1]non-9-yl]acetate;
(21) ethyl exo-4-[3-(4-acetylamino-5-chloro-2-methoxybenzamido)-9-azabicyclo[3.3.1]non-9-yl]butyrate;
(22) ethyl endo-[3-(4-acetylamino-5-chloro-2-methoxybenzamido)-9-azabicyclo[3.3.1]non-9-yl]acetate;
(23) ethyl endo-3-[3-(4-acetylamino-5-chloro-2-methoxybenzamido)-9-azabicyclo[3.3.1]non-9-yl]propionate;
(24) ethyl endo-4-[3-(4-acetylamino-5-chloro-2-methoxybenzamido)-9-azabicyclo[3,3,1]non-9-yl]butyrate;
(25) ethyl endo-5-[3-(4-acetylamino-5-chloro-2-methoxybenzamido)-9-azabicyclo[3.3.1]non-9-yl]valerate;
(26) methyl endo-6-[3-(4-acetylamino-5-chloro-2-methoxybenzamido)-9-azabicyclo[3.3.1]non-9-yl]hexanoate;
(27) methyl endo-7-[3-(4-acetylamino-5-chloro-2-methoxybenzamido)-9-azabicyclo[3.3.1]non-9-yl]heptanoate;
(28) methyl endo-8-[3-(4-acetylamino-5-chloro-2-methoxybenzamido)-9-azabicyclo[3.3.1]non-9-yl]octanoate;
(29) ethyl endo-[3-(4-acetylamino-5-chloro-2-ethoxybenzamido)-9-azabicyclo[3.3.1]non-9-yl]acetate;
(30) ethyl endo-4-[3-(4-acetylamino-5-chloro-2-ethoxybenzamido)-9-azabicyclo[3.3.1]non-9-yl]butyrate;
(31) ethyl exo-[3-(4-amino-5-chloro-2-methoxybenzamido)-8-azabicyclo[3.2.1]oct-8-yl]acetate;
(32) ethyl exo-4-[3-(4-amino-5-chloro-2-methoxybenzamido)-8-azabicyclo[3.2.1]oct-8-yl]butyrate;
(33) ethyl endo-[3-(4-amino-5-chloro-2-methoxybenzamido)-8-azabicyclo[3.2.1]oct-8-yl]acetate;
(34) ethyl endo-4-[3-(4-amino-5-chloro-2-methoxybenzamido)-8-azabicyclo[3.2.1]oct-8-yl]butyrate;
(35) ethyl exo-[3-(4-amino-5-chloro-2-methoxybenzamido)-9-azabicyclo[3.3.1]non-9-yl]acetate;
(36) ethyl exo-4-[3-(4-amino-5-chloro-2-methoxybenzamido)-9-azabicyclo[3.3.1]non-9-yl]butyrate;
(37) ethyl endo-[3-(4-amino-5-chloro-2-methoxybenzamido)-9-azabicyclo[3.3.1]non-9-yl]acetate;
(38) ethyl endo-3-[3-(4-amino-5-chloro-2-methoxybenzamido)-9-azabicyclo[3.3.1]non-9-yl]propionate;
(39) ethyl endo-2-[3-(4-amino-5-chloro-2-methoxybenzamido)-9-azabicyclo[3.3.1]non-9-yl]propionate;
(40) ethyl endo-4-[3-(4-amino-5-chloro-2-methoxybenzamido)-9-azabicyclo[3.3.1]non-9-yl]butyrate;
(41) ethyl endo-5-[3-(4-amino-5-chloro-2-methoxybenzamido)-9-azabicyclo[3.3.1]non-9-yl]valerate;
(42) ethyl endo-6-[3-(4-amino-5-chloro-2-methoxybenzamido)-9-azabicyclo[3.3.1]non-9-yl]hexanoate;
(43) methyl endo-7-[3-(4-amino-5-chloro-2-methoxybenzamido)-9-azabicyclo[3.3.1]non-9-yl]heptanoate;
(44) ethyl endo-8-[3-(4-amino-5-chloro-2-methoxybenzamido)-9-azabicyclo[3.3.1]non-9-yl]octanoate;
(45) ethyl endo-[3-(4-amino-5-chloro-2-ethoxybenzamido)-9-azabicyclo[3.3.1]non-9-yl]acetate;
(46) ethyl endo-4-[3-(4-amino-5-chloro-2-ethoxybenzamido)-9-azabicyclo[3.3.1]non-9-yl]butyrate;
(47) ethyl endo-[3-[(4-amino-5-chloro-2-methoxybenzamido)methyl]-9-azabicyclo[3.3.1]non-9-yl]acetate;
(48) ethyl endo-4-[3-[(4-amino-5-chloro-2-methoxybenzamido)methyl]-9-azabicyclo[3.3.1]non-9-yl]butyrate;
(49) ethyl endo-[3-[2-(4-amino-5-chloro-2-methoxybenzamido)ethyl]-9-azabicyclo[3.3.1]non-9-yl]acetate;
(50) ethyl endo-4-[3-[2-(4-amino-5-chloro-2-methoxybenzamido)ethyl]-9-azabicyclo[3.3.1]non-9-yl]butyrate;
(51) ethyl endo-[3-[3-(4-amino-5-chloro-2-methoxybenzamido)propyl]-9-azabicyclo[3.3.1]non-9-yl]acetate;
(52) ethyl endo-4-[3-[3-(4-amino-5-chloro-2-methoxybenzamido)propyl]-9-azabicyclo[3.3.1]non-9-yl]butyrate;
(53) exo-[3-(4-amino-5-chloro-2-methoxybenzamido)-8-azabicyclo[3.2.1]oct-8-yl]acetic acid;
(54) exo-4-[3-(4-amino-5-chloro-2-methoxybenzamido)-8-azabicyclo[3.2.1]oct-8-yl]butyric acid;
(55) endo-[3-(4-amino-5-chloro-2-methoxybenzamido)-8-azabicyclo[3.2.1]oct-8-yl]acetic acid;
(56) endo-4-[3-(4-amino-5-chloro-2-methoxybenzamido)-8-azabicyclo[3.2.1]oct-8-yl]butyric acid;
(57) exo-[3-(4-amino-5-chloro-2-methoxybenzamido)-9-azabicyclo[3.3.1]non-9-yl]acetic acid;
(58) exo-4-[3-(4-amino-5-chloro-2-methoxybenzamido)-9-azabicyclo[3.3.1]non-9-yl]butyric acid;
(59) endo-[3-(4-amino-5-chloro-2-methoxybenzamido)-9-azabicyclo[3.3.1]non-9-yl]acetic acid;
(60) endo-3-[3-(4-amino-5-chloro-2-methoxybenzamido)-9-azabicyclo[3.3.1]non-9-yl]propionic acid;
(61) endo-2-[3-(4-amino-5-chloro-2-methoxybenzamido)-9-azabicyclo[3.3.1]non-9-yl]propionic acid;
(62) endo-4-[3-(4-amino-5-chloro-2-methoxybenzamido)-9-azabicyclo[3.3.1]non-9-yl]butyric acid;
(63) endo-5-[3-(4-amino-5-chloro-2-methoxybenzamido)-9-azabicyclo[3.3.1]non-9-yl]valeric acid;
(64) endo-6-[3-(4-amino-5-chloro-2-methoxybenzamido)-9-azabicyclo[3.3.1]non-9-yl]hexanoic acid;
(65) endo-7-[3-(4-amino-5-chloro-2-methoxybenzamido)-9-azabicyclo[3.3.1]non-9-yl]heptanoic acid;
(66) endo-8-[3-(4-amino-5-chloro-2-methoxybenzamido)-9-azabicyclo[3.3.1]non-9-yl]octanoic acid;
(67) endo-[3-(4-amino-5-chloro-2-ethoxybenzamido)-9-azabicyclo[3.3.1]non-9-yl]acetic acid;
(68) endo-4-[3-(4-amino-5-chloro-2-ethoxybenzamido)-9-azabicyclo[3.3.1]non-9-yl]butyric acid;
(69) endo-[3-[(4-amino-5-chloro-2-methoxybenzamido)methyl]-9-azabicyclo[3.3.1]non-9-yl]acetic acid;
(70) endo-4-[3-[(4-amino-5-chloro-2-methoxybenzamido)methyl]-9-azabicyclo[3.3.1]non-9-yl]butyric acid;
(71) endo-[3-[2-(4-amino-5-chloro-2-methoxybenzamido)ethyl]-9-azabicyclo[3.3.1]non-9-yl]acetoc acid;
(72) endo-4-[3-[2-(4-amino-5-chloro-2-methoxybenzamido)ethyl]-9-azabicyclo[3.3.1]non-9-yl]butyric acid;
(73) endo-[3-[3-(4-amino-5-chloro-2-methoxybenzamido)propyl]-9-azabicyclo[3.3.1]non-9-yl]acetic acid;
(74) endo-4-[3-[3-(4-amino-5-chloro-2-methoxybenzamido)propyl]-9-azabicyclo[3.3.1]non-9-yl]butyric acid;
(75) ethyl [4-(4-amino-5-chloro-2-methoxybenzamido)piperidino]acetate;
(76) ethyl 2-[4-(4-amino-5-chloro-2-methoxybenzamido)piperidino]propionate;
(77) ethyl 3-[4-(4-amino-5-chloro-2-methoxybenzamido)piperidino]propionate;
(78) ethyl 4-[4-(4-amino-5-chloro-2-methoxybenzamido)piperidino]butyrate;
(79) ethyl 5-[4-(4-amino-5-chloro-2-methoxybenzamido)piperidino]valerate;
(80) methyl 6-[4-(4-amino-5-chloro-2-methoxybenzamido)piperidino]hexanoate;
(81) ethyl [4-[(4-amino-5-chloro-2-methoxybenzamido)methyl]piperidino]acetate;
(82) ethyl 4-[4-[(4-amino-5-chloro-2-methoxybenzamido)methyl]piperidino]butyrate;
(83) ethyl [4-[2-(4-amino-5-chloro-2-methoxybenzamido)ethyl]piperidino]acetate;
(84) ethyl 4-[4-[2-(4-amino-5-chloro-2-methoxybenzamido)ethyl]piperidino]butyrate;
(85) ethyl [4-[3-(4-amino-5-chloro-2-methoxybenzamido)propyl]piperidino]acetate;
(86) ethyl 4-[4-[3-(4-amino-5-chloro-2-methoxybenzamido)propyl]piperidino]butyrate;
(87) [4-(4-amino-5-chloro-2-methoxybenzamido)piperidino]acetic acid;
(88) 2-[4-(4-amino-5-chloro-2-methoxybenzamido)piperidino]propionic acid;
(89) 3-[4-(4-amino-5-chloro-2-methoxybenzamido)piperidino]propionic acid;
(90) 4-[4-(4-amino-5-chloro-2-methoxybenzamido)piperidino]butyric acid;
(91) 5-[4-(4-amino-5-chloro-2-methoxybenzamido)piperidino]valeric acid;
(92) 6-[4-(4-amino-5-chloro-2-methoxybenzamido)piperidino]hexanoic acid;
(93) [4-[(4-amino-5-chloro-2-methoxybenzamido)methyl]piperidino]acetic acid;
(94) 4-[4-[(4-amino-5-chloro-2-methoxybenzamido)methyl]piperidino]butyric acid;
(95) [4-[2-(4-amino-5-chloro-2-methoxybenzamido)ethyl]piperidino]acetic acid;
(96) 4-[4-[2-(4-amino-5-chloro-2-methoxybenzamido)ethyl]piperidino]butyric acid;
(97) [4-[3-(4-amino-5-chloro-2-methoxybenzamido)propyl]piperidino]acetic acid;
(98) 4-[4-[3-(4-amino-5-chloro-2-methoxybenzamido)propyl]piperidino]butyric acid;
(99) ethyl cis-[4-(4-amino-5-chloro-2-methoxybenzamido)-3-methoxypiperidino]acetate;
(100) ethyl trans-[4-(4-amino-5-chloro-2-methoxybenzamido)-3-methoxypiperidino]acetate;
(101) ethyl cis-4-[4-(4-amino-5-chloro-2-methoxybenzamido)-3-methoxypiperidino]butyrate;
(102) ethyl trans-4-[4-(4-amino-5-chloro-2-methoxybenzamido)-3-methoxypiperidino]butyrate;
(103) methyl cis-6-[4-(4-amino-5-chloro-2-methoxybenzamido)-3-methoxypiperidino]hexanoate;
(104) ethyl cis-[4-(4-amino-5-chloro-2-methoxybenzamido)-3-methylpiperidino]acetate;
(105) ethyl trans-[4-(4-amino-5-chloro-2-methoxybenzamido)-3-methylpiperidino]acetate;
(106) ethyl cis-4-[4-(4-amino-5-chloro-2-methoxybenzamido)-3-methylpiperidino]butyrate;
(107) ethyl trans-4-[4-(4-amino-5-chloro-2-methoxybenzamido)-3-methylpiperidino]butyrate;
(108) methyl cis-6-[4-(4-amino-5-chloro-2-methoxybenzamido)-3-methylpiperidino]hexanoate;
(109) cis-[4-(4-amino-5-chloro-2-methoxybenzamido)-3-methoxypiperidino]acetic acid;
(110) trans-[4-(4-amino-5-chloro-2-methoxybenzamido)-3-methoxypiperidino]acetic acid;
(111) cis-4-[4-(4-amino-5-chloro-2-methoxybenzamido)-3-methoxypiperidino]butyric acid;
(112) trans-4-[4-(4-amino-5-chloro-2-methoxybenzamido)-3-methoxypiperidino]butyric acid;
(113) cis-6-[4-(4-amino-5-chloro-2-methoxybenzamido)-3-methoxypiperidino]hexanoic acid;
(114) cis-[4-(4-amino-5-chloro-2-methoxybenzamido)-3-methylpiperidino]butyric acid;
(115) trans-[4-(4-amino-5-chloro-2-methoxybenzamido)-3-methylpiperidino]acetic acid;
(116) cis-4-[4-(4-amino-5-chloro-2-methoxybenzamido)-3-methylpiperidino]butyric acid;
(117) trans-4-[4-(4-amino-5-chloro-2-methoxybenzamido)-3-methylpiperidino]butyric acid;
(118) cis-6-[4-(4-amino-5-chloro-2-methoxybenzamido)-3-methylpiperidino]hexanoic acid;
(119) ethyl [2-[(4-amino-5-chloro-2-methoxybenzamido)methyl]morpholino]acetate;
(120) ethyl 4-[2-[(4-amino-5-chloro-2-methoxybenzamido)methyl]morpholino]butyrate;
(121) [2-[(4-amino-5-chloro-2-methoxybenzamido)methyl]morpholino]acetic acid;
(122) 4-[2-[(4-amino-5-chloro-2-methoxybenzamido)methyl]morpholino]butyric acid;
(123) ethyl [2-[(4-amino-5-chloro-2-methoxybenzamido)methyl]thiamorpholino]acetate;
(124) ethyl 4-[2-[(4-amino-5-chloro-2-methoxybenzamido)methyl]thiamorpholino]butyrate;
(125) [2-[(4-amino-5-chloro-2-methoxybenzamido)methyl]thiamorpholino]acetic acid;
(126) 4-[2-[(4-amino-5-chloro-2-methoxybenzamido)methyl]thiamorpholino]butyric acid;
(127) ethyl [3-[(4-amino-5-chloro-2-methoxybenzamido)methyl]pyrrolidin-1-yl]acetate;
(128) ethyl 4-[3-[(4-amino-5-chloro-2-methoxybenzamido)methyl]pyrrolidin-1-yl]butyrate;
(129) [3-[(4-amino-5-chloro-2-methoxybenzamido)methyl]pyrrolidin-1-yl]acetic acid;
(130) 4-[3-[(4-amino-5-chloro-2-methoxybenzamido)methyl]pyrrolidin-1-yl]butyric acid;
(131) ethyl [3-(4-amino-5-chloro-2-methoxybenzamido)pyrrolidin-1-yl]acetate;
(132) ethyl 4-[3-(4-amino-5-chloro-2-methoxybenzamido)pyrrolidin-1-yl]butyrate;
(133) [3-(4-amino-5-chloro-2-methoxybenzamido)pyrrolidin-1-yl]acetic acid; and
(134) 4-[3-(4-amino-5-chloro-2-methoxybenzamido)pyrrolidin-1-yl]butyric acid. However, the present invention is not limited to these examples.

The benzamide derivative represented by formula (I) can be prepared according to a method described below which is an embodiment of the present invention. However, methods for preparing said compound are not limited to these processes.

A process for preparing the benzamide derivatives of the present invention represented by formula (I) comprises the following steps:
(a) reacting a compound represented by the following formula (II) wherein R¹, R², R³, R⁵, X, m, n, and p are the same as those defined above: 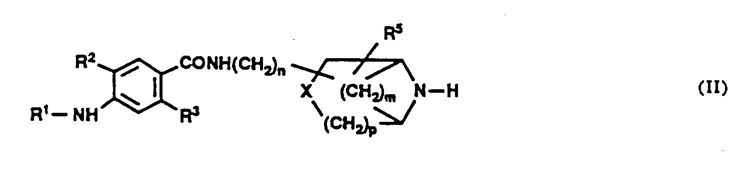 with a compound represented by the following formula (III) wherein A is the same as that defined above, R⁶ represents a C₁-C₄ alkyl, and Y represents a halogen atom:

   Y-A-CO₂ R⁶ or CH=CH₂CO₂R⁶ (III)

   to carry out N-alkylation reaction in an organic solvent or without a solvent and in the presence or absence of a base as a dehydrohalogenating agent; and
(b) halogenating or hydrolyzing the product obtained in the above step (a), if desired.

Examples of the organic solvent used for the N-alkylation in the method of the present invention include, for example, alcohols such as, for example, methanol, ethanol, n-propanol, isopropanol, and n-butanol; aromatic hydrocarbons such as, for example, benzene, toluene, and xylene; and aprotic polar solvents such as, for example, tetrahydrofuran, 1,4-dioxane, acetonitrile, N,N-dimethylformamide, N-methyl-2-pyrrolidone, and dimethyl sulfoxide. Examples of the base used include, for example, metallic sodium, sodium hydride, sodium methoxide, sodium ethoxide, sodium amide, sodium hydroxide, potassium hydroxide, sodium carbonate, potassium carbonate, sodium hydrogen carbonate, and potassium hydrogen carbonate. The reaction may be carried out at from ice-cooling temperature to refluxing temperature of a solvent.

Halogenation may be carried out by the treatment with a halogenating agent in an organic solvent. Examples of the halogenating agent used include, for example, chlorine, bromine, and sulfuryl chloride. Examples of the organic solvent used include, for example, alcohols such as, for example, methanol, ethanol, and n-propanol; halogenated hydrocarbons such as, for example, dichloromethane, chloroform, carbon tetrachloride, and 1,2-dichloroethane; and aliphatic acids such as, for example, acetic acid. The reaction may be carried out at from -30°C to refluxing temperature of a solvent.

Hydrolysis may be carried out by using an acid or a base. For acidic hydrolysis, acids such as hydrochloric acid or sulfric acid may be used, and for alkaline hydrolysis, bases such as sodium hydroxide, potassium hydroxide, sodium carbonate, or potassium carbonate may be used. These acids and bases may be used in the reaction in a form of aqueous solution, or alternatively, solutions in methanol, ethanol, n-propanol, isopropanol, n-butanol, sec-butanol, or tert-butanol, or solutions in aqueous organic solvents. The reaction may be carried out at from ice-cooling temperature to refluxing temperature of a solvent.

The compounds represented by formula (II) used as starting materials in the aforementioned preparation process of the present invention are novel compounds with a few exceptions. The compounds can be prepared according to the scheme set out below, in which R¹, R², R³, R⁵, X, m, n, and p are the same as those defined above, R⁷ represents a C₁-C₄ alkyl group, and Z represents a halogen atom. Specific examples of the preparation process are given as reference examples in Examples which follows.

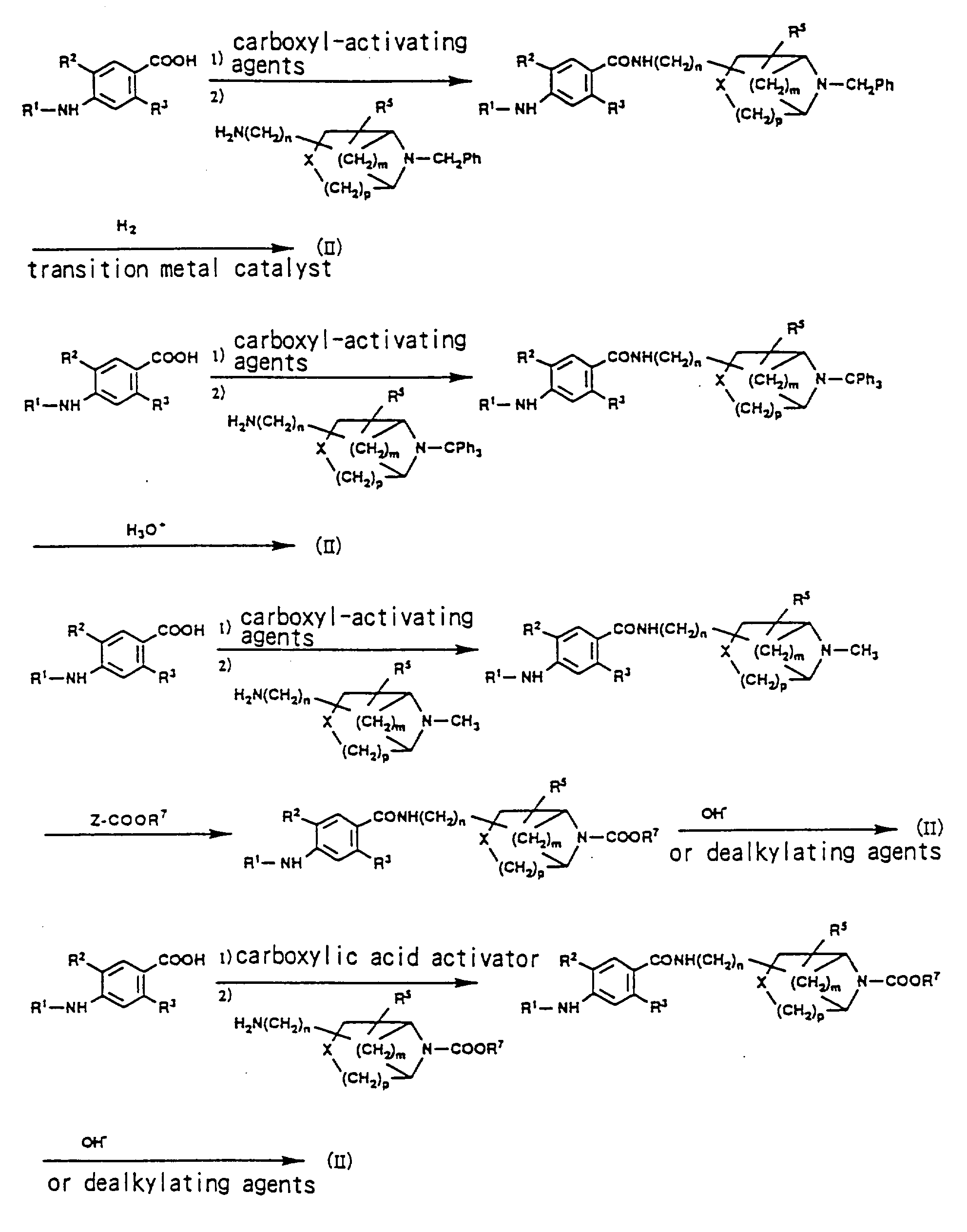

The benzamide derivatives represented by formula (I) prepared according to the process described above and their pharmacologically acceptable salts have hyderfunctional activity on digestive tract and are useful for the treatment of gastrointestinal diseases. The compounds may preferably be formulated in a pharmaceutical composition as an active ingredient. The pharmaceutical composition comprising said compound as an active ingredient may generally be formulated and administered to a patient as orally available compositions such as, for example, capsules, tablets, subtilized granules, granules, powder or syrup, or administered as injection, suppository, eye drop, eye ointment, ear drop, or topical composition.

These pharmaceutical compositions can be prepared by ordinary methods. If necessary, pharmacologically and pharmaceutically acceptable additives may be added. For the preparation of orally available compositions and suppository, excipients such as, for example, lactose, D-mannitol, cornstarch, or crystalline cellulose; disintegrants such as, for example, carboxymethylcellulose or calcium carboxymethylcellulose; binders such as, for example, hydroxypropylcellulose, hydroxypropylmethylcellulose, or polyvinylpyrrolidone; lubricants such as, for example, magnesium stearate or talc; coating agents such as, for example, hydroxypropylmethylcellulose, sucrose, or titanium oxide; or bases such as, for example polyethylene glycol or hard fat may be used as pharmaceutical additives. For the preparation of an injection, eye drop, or ear drop, solubilizing agents or solubilizers such as, for example, distilled water for injection, saline, or propylene glycol which is useful for an aqueous composition or a composition for preparing aqueous solution before use; pH adjusting agents such as, for example, an inorganic or organic acid or base; isotonicity agents such as, for example, sodium chloride, glucose, or glycerin; or stabilizers may be used as pharmaceutical additives. For the preparation of eye ointment and topical composition, suitable pharmaceutical additives ordinarily formulated in ointment, cream, or cataplasms such as white vaseline, macrogol, glycerin, or cloth may be used.

The pharmaceutical compositions of the present invention may be administered orally to an adult patient at a daily dose of about 0.1 to 500 mg. The dose may be increased or decreased depending on the age or conditions of a patient.

As an example demonstrating remarkable advantageous effects of the compounds of the present invention, experimental results are set out below relating to effects on motility of digestive tract of a conscious dog.

### Effects on gastrointestinal motility in conscious dog

Under systemical anesthesia, force transducers were chronically implanted to the stomach and the colon of the adult male or female dogs in a direction to measure circular muscle contraction according to the method of Ito et al. (Japanese Journal of Smooth Muscle Research, 13, 33, 1976). A silicone tube chronically attached into jugular vein for intravenous administration of test compounds. After the animal was recovered from the operation, test compounds were administered at about two hours after feeding while gastrointestinal motility was measured. The gastrointestinal motility was indicated as motor index, and the effects of test compounds were estimated by the change of motor index for twenty minutes before and after the administration. The results are summarized in Table 1. It can be concluded that the compounds of the present invention exhibit excellent stimulating effects on lower digestive tracts as well as upper digestive tracts.

**Table 1**

| Stimulating Effect on Gastrointestinal Motility (Conscious Dog) | | | |
|---|---|---|---|
| Test Compound | Dose | Change of Motor Index (%) | |
| | (mg/kg, i.v.) | Stomach | Colon |
| Example 23 | 1 | 133.5 | 133.3 |
| Example 24 | 0.1 | 116.0 | 134.8 |
| Example 25 | 1 | 180.9 | 132.7 |
| Example 27 | 0.1 | 113.4 | 134.5 |
| Example 28 | 1 | 141.5 | 163.7 |
| Example 32 | 1 | 176.7 | 158.5 |
| Example 33 | 1 | 147.1 | 117.4 |
| Example 34 | 1 | 187.0 | 191.5 |
| Example 35 | 1 | 186.8 | 135.8 |
| Example 36 | 1 | 195.9 | 119.6 |
| Example 38 | 1 | 145.6 | 140.3 |
| Example 39 | 0.1 | 134.7 | 159.1 |
| Example 41 | 1 | 118.3 | 172.0 |
| Example 43 | 1 | 166.5 | 504.8 |
| Example 46 | 1 | 182.5 | 165.6 |
| Example 58 | 0.1 | 180.7 | 217.5 |
| Example 63 | 1 | 129.6 | 156.6 |
| Example 64 | 0.1 | 200.3 | 234.5 |

The present invention will be explained by way of reference examples and examples. However, the present invention is not limited to these examples.

### EXAMPLES

### Reference Example 1: endo-4-Acetylamino-N-(9-azabicyclo[3.3.1]non-3-yl)-2-methoxybenzamide

(1) endo-4-Acetylamino-N-(9-benzyl-9-azabicyclo[3.3.1]non-3-yl)-2-methoxybenzamide
To a mixture of 11.0 g of 4-acetylamino-2-methoxybenzoic acid, 8.5 ml of triethylamine, and 210 ml of tetrahydrofuran, 5.3 ml of ethyl chloroformate was added dropwise with stirring under ice-cooling. After the mixture was stirred for 1.5 hours under ice-cooling, 12.72 g of endo-9-benzyl-9-azabicyclo[3.3.1]inonan-3-amine was added by portions. Stirring was continued for 2.5 days at room temperature, and then the mixture was concentrated under reduced pressure. Water was added to the residue, and pH was adjusted to 9 using 10% aqueous potassium carbonate solution. Crystals precipitated were collected by filtration and washed with water and then with ethanol to give 15.7 g of colorless crystals. Recrystallization from methanol gave colorless needles, m.p. 233.5-235 °C .

| Anal. C₂₅ H₃₁ N₃ O₃ | | | |
|---|---|---|---|
| Calcd. | C: 71.23; | H: 7.41; | N: 9.97 |
| Found | C: 71.19; | H: 7.50; | N: 9.93 |

(2) endo-4-Acetylamino-N-(9-azabicyclo[3.3.1]non-3-yl)-2-methoxybenzamide
To a mixture of 15.5 g of endo-4-acetylamino-N-(9-benzyl-9-azabicyclo[3.3.1]non-3-yl)-2-methoxybenzamide, 20 ml of acetic acid, and 180 ml of methanol, 2.0 g of Pearlman's catalyst (20% palladium hydroxide on carbon) was added, and then hydrogenolysis was carried out for 1 hour at room temperature under ordinary pressure. The catalyst was removed and the filtrate was concentrated under reduced pressure. The residue was taken up in water and pH was adjusted to 9 by adding potassium carbonate. Crystals precipitated were collected by filtration and washed with water to give 12.6 g of pale yellow crystals. Recrystallization from 1,2-dichloroethane gave colorless prisms, m.p. 196-200 °C .

| Anal. C₁₈H₂₅N₃O₃ · 1/4H₂O | | | |
|---|---|---|---|
| Calcd. | C: 64.36; | H: 7.65; | N: 12.51 |
| Found | C: 64.32; | H: 7.53; | N: 12.47 |

In the same manner as Reference Example 1, the compounds of Reference Examples 2-4 were obtained.

### Reference Example 2: exo-4-Acetylamino-N-(8-azabicyclo[3.2.1]oct-3-yl)-2-methoxybenzamide fumarate

(1) exo-4-Acetylamino-N-(8-benzyl-8-azabicyclo[3.2.1]oct-3-yl)-2-methoxybenzamide
Appearance: pale brown needles (MeOH)
IR Spectrum ν (KBr) cm⁻¹ : 1694, 1668
NMR Spectrum δ (DMSO-d₆) ppm : 1.50-1.83(6H,m), 1.88-2.14(2H,m), 2.06(3H,s), 3.05-3.32(2H,m), 3.55(2H,s), 3.85(3H,s), 4.05-4.29(1H,m), 7.10-7.45(5H,m), 7.17(1H,dd,J=8.6,1.7Hz), 7.48(1H,d,J=1.7Hz), 7.62-7.77(1H,m), 7.70(1H,d,J=8.6Hz), 10.04(1H,s)
Mass Spectrum m/z : 407 (M⁺)
(2) exo-4-Acetylamino-N-(8-azabicyclo[3.2.1]oct-3-yl)-2-methoxybenzamide fumarate
Appearance: colorless crystals (MeOH)
m.p. 223-225°C (decomp.)

| Anal. C₁₇H₂₃N₃O₃ · C₄H₄O₄ | | | |
|---|---|---|---|
| Calcd. | C: 58.19; | H: 6.28; | N: 9.69 |
| Found | C: 58.05; | H: 6.45; | N: 9.71 |

### Reference Example 3: exo-4-Acetylamino-N-(9-azabicyclo[3.3.1]non-3-yl)-2-methoxybenzamide fumarate

(1) exo-4-Acetylamino-N-(9-benzyl-9-azabicyclo[3.3.1]non-3-yl)-2-methoxybenzamide
Appearance: slightly brown needles (MeOH)
m.p. 215-216°C

| Anal. C₂₅H₃₁N₃O₃ | | | |
|---|---|---|---|
| Calcd. | C: 71.23; | H: 7.41; | N: 9.97 |
| Found | C: 71.12; | H: 7.48; | N: 9.89 |

(2) exo-4-Acetylamino-N-(9-azabicyclo[3.3.1]non-3-yl)-2-methoxybenzamide fumarate
Appearance: colorless crystals (MeOH)
m.p. 194-196°C (decomp.)

| Anal. C₁₈H₂₅N₃O₃ · C₄H₄O₄ · 3/4H₂O | | | |
|---|---|---|---|
| Calcd. | C: 57.32; | H: 6.67; | N: 9.12 |
| Found | C: 57.13; | H: 6.58; | N: 9.12 |

### Reference Example 4: endo-4-Acetylamino-N-(9-azabicyclo[3.3.1]non-3-yl)-2-ethoxybenzamide

(1) endo-4-Acetylamino-N-(9-benzyl-9-azabicyclo[3.3.1]non-3-yl)-2-ethoxybenzamide
Appearance: colorless needles (MeOH)
m.p. 215-216°C

| Anal. C₂₆H₃₃N₃O₃ | | | |
|---|---|---|---|
| Calcd. | C: 71.70; | H: 7.64; | N: 9.65 |
| Found | C: 71.45; | H: 7.61; | N: 9.47 |

(2) endo-4-Acetylamino-N-(9-azabicyclo[3.3.1]non-3-yl)-2-ethoxybenzamide
Appearance: colorless needles (H₂O)
m.p. 116-119°C
224-226°C

| Anal. C₁₉H₂₇N₃O₃ · 5/2H₂O | | | |
|---|---|---|---|
| Calcd. | C: 58.44; | H: 8.26; | N: 10.76 |
| Found | C: 58.45; | H: 7.86; | N: 10.40 |

### Reference Example 5: endo-4-Acetylamino-N-(8-azabicyclo[3.2.1]oct-3-yl)-5-chloro-2-methoxybenzamide

(1) endo-4-Acetylamino-5-chloro-2-methoxy-N-(8-methyl-8-azabicyclo[3.2.1]oct-3-yl)benzamide
To a mixture of 38.0 g of 4-acetylamino-5-chloro-2-methoxybenzoic acid, 26.1 ml of triethylamine, and 500 ml of tetrahydrofuran, 15.7 ml of ethyl chloroformate was added dropwise with stirring under ice-cooling. Stirring was continued for 1 hour under ice-cooling, and then a solution of 23.0 g of endo-8-methyl-8-azabicyclo[3.2.1]octan-3-amine in 40 ml of tetrahydrofuran was added dropwise. Stirring was continued for 1.5 hours at room temperature, and then insoluble materials were removed and the filtrate obtained was concentrated under reduced pressure. Water was added to the residue and pH was adjusted to 9 by using aqueous potassium carbonate solution. Crystals precipitated were collected by filtration and washed with water and then with ethyl acetate to give 48.6 g of slightly yellow crystals.
(2) Methyl endo-[3-(4-acetylamino-5-chloro-2-methoxybenzamido)-8-azabicyclo[3.2.1]oct-8-yl]formate
To a mixture of 40.0 g of endo-4-acetylamino-5-chloro-2-methoxy-N-(8-methyl-8-azabicyclo[3.2.1]oct-3-yl)benzamide, 16.0 g of potassium carbonate, and 400 ml of chloroform, 80.0 ml of methyl chloroformate was added dropwise with stirring at room temperature. The mixture was refluxed for 19 hours, and then insoluble materials were removed and the filtrate obtained was concentrated under reduced pressure. The residue was washed with ethyl acetate and then with hot methanol to give 17.5 g of colorless crystals. Recrystallization from dichloromethane-methanol gave colorless needles, m.p. 250-252°C.

| Anal. C₁₉H₂₄ClN₃O₅ · 1/2H₂O | | | |
|---|---|---|---|
| Calcd. | C: 54.48; | H: 6.02; | N: 10.03 |
| Found | C: 54.17; | H: 5.77; | N: 9.79 |

(3) endo-4-Acetylamino-N-(8-azabicyclo[3.2.1]oct-3-yl)-5-chloro-2-methoxybenzamide
To a suspension of 16.3 g of methyl endo-[3-(4-acetylamino-5-chloro-2-methoxybenzamido)-8-azabicyclo[3.2.1]oct-8-yl]formate in 400 ml of dichloromethane, 22.0 ml of iodo trimethylsilane was added dropwise with stirring at room temperature. Stirring was continued for 6 hours at room temperature, and then aqueous sodium hydrosulfite solution was added. Aqueous layer was separated after pH was adjusted to 2 using 10% hydrochloric acid. The aqueous layer was basified using potassium carbonate and then extracted with dichloromethane. The extract was dried over anhydrous sodium sulfate and concentrated under reduced pressure. The residue was purified using column chromatography on aluminum oxide [eluent: dichloromethane→ dichloromethane-methanol (50:1)] to afford 8.41 g of pale yellow syrup.
IR Spectrum ν (liq) cm⁻¹ : 1694, 1644
NMR Spectrum δ (CDCl₃) ppm : 1.65-2.30(9H,m), 2.28(3H,s), 3.60-3.74(2H,m), 4.02(3H,s), 4.30-4.46(1H,m), 7.82(1H,br-s), 8.21(1H,s), 8.34(1H,s), 8.40-8.50(1H,m)

| High Resolution Mass Spectrum : C₁₇ H₂₂ ClN₃ O₃ | | | |
|---|---|---|---|
| Calcd. | m/z : | 351.1350, | 353.1320 |
| Found | m/z : | 351.1363, | 353.1325 |

In the same manner as Reference Example 5, the compound of Reference Example 6 was obtained.

### Reference Example 6: endo-4-Acetylamino-N-(9-azabicyclo[3.3.1]non-3-yl)-5-chloro-2-methoxybenzamide

(1) endo-4-Acetylamino-5-chloro-2-methoxy-N-(9-methyl-9-azabicyclo[3.3.1]non-3-yl)benzamide
Appearance: slightly brown amorphous solid
IR Spectrum ν (KBr) cm⁻¹ : 1706, 1646
NMR Spectrum δ (CDCl₃) ppm: 1.00-1.15(2H,m), 1.20-1.40(2H,m), 1.45-1.60(1H,m), 1.85-2.10(3H,m), 2.28(3H,s), 2.40-2.75(2H,m), 2.50(3H,s), 3.00-3.15(2H,m), 3.98(3H,s), 4.35-4.60(1H,m), 7.60-7.70(1H,m), 7.82(1H,br-s), 8.21(1H,s), 8.30(1H,s)

| High Resolution Mass Spectrum: C₁₉ H₂₆ ClN₃ O₃ | | | |
|---|---|---|---|
| Calcd. | m/z : | 379.1663, | 381.1633 |
| Found | m/z : | 379.1654, | 381.1630 |

(2) Methyl endo-[3-(4-acetylamino-5-chloro-2-methoxybenzamido)-9-azabicyclo[3.3.1]non-9-yl)formate
Appearance: colorless crystals (AcOEt)
m.p. 202-205°C

| Anal. C₂₀ H₂₆ ClN₃ O₅ | | | |
|---|---|---|---|
| Calcd. | C: 56.67; | H: 6.18; | N: 9.91 |
| Found | C: 56.49; | H: 6.25; | N: 9.69 |

(3) endo-4-Acetylamino-N-(9-azabicyclo[3.3.1]non-3-yl)-5-chloro-2-methoxybenzamide
Appearance: pale yellow syrup
IR Spectrum ν (liq) cm⁻¹ : 1694, 1646
NMR Spectrum δ (CDCl₃) ppm: 1.14-2.00(8H,m), 2.18(1H,br-s), 2.28(3H,s), 2.20-2.40(2H,m), 3.30-3.47(2H,m), 3.99(3H,s), 4.06-4.28(1H,m), 7.57-7.70(1H,m), 7.81(1H,br-s), 8.22(1H,s), 8.32(1H,s)

| High Resolution Mass Spectrum: C₁₈ H₂₄ ClN₃ O₃ | | | |
|---|---|---|---|
| Calcd. | m/z : | 365.1506, | 367.1477 |
| Found | m/z : | 365.1510, | 367.1457 |

### Reference Example 7: endo-4-Amino-N-(8-azabicyclo[3.2.1]oct-3-yl)-5-chloro-2-methoxybenzamide hydrochloride

To a solution of 7.86 g of endo-4-acetylamino-N-(8-azabicyclo[3.2.1]oct-3-yl)-5-chloro-2-methoxybenzamide in 23 ml of ethanol, 40 ml of 32% hydrogen chloride/ethanol solution was added and then the mixture was refluxed for 5.5 hours. The reaction mixture was concentrated under reduced pressure, and the residue was washed with ethanol to give 5.05 g of pale brown crystals. Recrystallization from methanol gave colorless needles, m.p. 280-282 °C (decomp.).

| Anal. C₁₅ H₂₀ ClN₃ O₂ · HCl · 3/4H₂O | | | |
|---|---|---|---|
| Calcd. | C: 50.08; | H: 6.30; | N: 11.68 |
| Found | C: 50.07; | H: 6.20; | N: 11.57 |

In the same manner as Reference Example 7, the compound of Reference Example 8 was obtained.

### Reference Example 8: endo-4-Amino-N-(9-azabicyclo[3.3.1]non-3-yl)-5-chloro-2-methoxybenzamide hydrochloride

Appearance: slightly brown needles (EtOH)
m.p. 257-261°C (decomp.)

| Anal. C₁₆H₂₂ClN₃O₂ · HCl · H₂O | | | |
|---|---|---|---|
| Calcd. | C: 50.80; | H: 6.66; | N: 11.11 |
| Found | C: 50.74; | H: 6.57; | N: 10.91 |

### Reference Example 9: 4-Amino-5-chloro-2-methoxy-N-(4-piperidinyl)benzamide hydrochloride

(1) 4-Amino-5-chloro-2-methoxy-N-(1-triphenylmethyl-4-piperidinyl)benzamide
   To a mixture of 16.40 g of 4-amino-5-chloro-2-methoxybenzoic acid, 13.60 ml of triethylamine, and 330 ml of dry tetrahydrofuran, 8.55 ml of ethyl chloroformate was added with stirring under ice-cooling. Stirring was continued for 2 hours under ice-cooling, 30.64 g of 4-amino-1-triphenylmethylpiperidine was added by portions. After stirring was continued for 20 hours at room temperature, insoluble materials were removed and the filtrate obtained was concentrated under reduced pressure. The residue was dissolved in dichloromethane and washed with water. The solution was dried over anhydrous sodium sulfate and then concentrated under reduced pressure. The residue was washed with n-hexane to give 39.42 g of colorless crystals.
   IR Spectrum ν (KBr) cm⁻¹ : 1646, 1624
   NMR Spectrum δ (CDCl₃) ppm: 1.57-1.81(2H,m), 1.92-2.19(2H,m), 2.73-3.38(2H,m), 3.58-4.08(3H,m), 4.33(2H,br-s), 6.25(1H,s), 7.05-7.55(15H,m), 7.67(1H,br-s), 8.06(1H,s)
(2) 4-Amino-5-chloro-2-methoxy-N-(4-piperidinyl)benzamide hydrochloride
   A mixture of 39.42 g of 4-amino-5-chloro-2-methoxy-N-(1-triphenylmethyl-4-piperidinyl)benzamide, 10 ml of concentrated hydrochloric acid, and 600 ml of acetone was refluxed for 40 minutes. After the mixture was cooled to 5 °C, crystals precipitated were collected by filtration and washed with acetone to give 25.79 g of colorless crystals, m.p. 165-168°C.
   IR Spectrum ν (KBr) cm⁻¹ : 2948, 2812, 1640
   NMR Spectrum δ (DMSO-d₆) ppm: 1.63-1.85(2H,m), 1.92-2.13(2H,m), 2.88-3.09(2H,m), 3.13-3.33(2H,m), 3.83(3H,s), 3.88-4.07(1H,m), 6.53(1H,s), 7.62(1H,s), 7.66-7.79(1H,m)
   Mass Spectrum m/z : 283, 285 (3:1) [M⁺ (free base)]

In the same manner as Reference Example 9, the compounds of Reference Examples 10 and 11 were obtained.

### Reference Example 10: 4-Amino-5-chloro-2-methoxy-N-[(2-morpholinyl)methyl]benzamide hydrochloride

(1) 4-Amino-5-chloro-2-methoxy-N-[(4-triphenylmethyl-2-morpholinyl)methyl]benzamide
Appearance: slightly brown crystals
NMR Spectrum δ (CDCl₃) ppm: 1.35-1.80(2H,m), 2.80-3.10(2H,m), 3.10-3.35(1H,m), 3.50-3.75(1H,m), 3.71(3H,s), 3.75-4.10(3H,m), 4.35(2H,br-s), 6.23(1H,s), 7.05-7.60(15H,m), 7.80-7.90(1H,m), 8.07(1H,s)
(2) 4-Amino-5-chloro-2-methoxy-N-[(2-morpholinyl)methyl]benzamide hydrochloride
Appearance: slightly brown crystals (H₂O)
m.p. 219-222°C

| Anal. C₁₃H₁₈ClN₃O₃ · HCl · H₂O | | | |
|---|---|---|---|
| Calcd. | C: 44.08; | H: 5.98; | N: 11.86 |
| Found | C: 44.20; | H: 5.88; | N: 11.82 |

### Reference Example 11: 4-Amino-5-chloro-2-methoxy-N-[(3-pyrrolidinyl) -methyl]benzamide hydrochloride

(1) 4-Amino-5-chloro-2-methoxy-N-[(1-triphenylmethyl-3-pyrrolidinyl) -methyl]benzamide
Appearance: slightly brown crystals
NMR Spectrum δ (CDCl₃) ppm : 1.35-1.80(2H,m), 1.50-1.65(1H,m), 1.76-1.96(1H,m), 2.10-2.40(3H,m), 2.46-2.50(1H,m), 3.30-3.60(2H,m), 3.77(3H,s), 4.35(2H,br-s), 6.26(1H,s), 7.05-7.60(15H,m), 7.60-7.75(1H,m), 8.10(1H,s)
(2) 4-Amino-5-chloro-2-methoxy-N-[(3-pyrrolidinyl)methyl]benzamide hydrochloride
Appearance: pale yellow crystals (H₂O)
m.p. 204-206°C

| Anal. C₁₃ H₁₈ ClN₃ O₂ · HCl · 5/4H₂O | | | |
|---|---|---|---|
| Calcd. | C: 45.56; | H: 6.32; | N: 12.26 |
| Found | C: 45.60; | H: 6.14; | N: 12.30 |

### Reference Example 12: cis-4-Amino-5-chloro-2-methoxy-N-[(3-methoxy-4-piperidinyl)benzamide

(1) Ethyl cis-[4-(4-amino-5-chloro-2-methoxybenzamido)-3-methoxypiperidino]formate
To a mixture of 33.3 g of 4-amino-5-chloro-3-methoxybenzoic acid, 27.0 ml of triethylamine, and 450 ml of tetrahydrofuran, 16.2 ml of ethyl chloroformate was added dropwise with stirring under ice-cooling. Stirring was continued for 1.5 hours under ice-cooling, a solution of 35.0 g of ethyl cis-(4-amino-3-methoxypiperidino)formate in 50 ml of tetrahydrofuran was added dropwise. After being stirred for 20 hours at room temperature, the reaction mixture was concentrated under reduced pressure. Water was added to the residue, and the mixture was extracted with dichloromethane. The extract was washed with aqueous potassium carbonate solution, dried over anhydrous sodium sulfate, and then concentrated under reduced pressure. The residue was washed with diethyl ether to give 44.5 g of colorless crystals.
(2) cis-4-Amino-5-chloro-2-methoxy-N-[(3-methoxy-4-piperidinyl) benzamide
A mixuture of 44.0 g of ethyl cis-[4-(4-amino-5-chloro-2-methoxybenzamide)-3-methoxypiperidino]formate, 67.7 g of potassium hydroxide, and 400 ml of iso-propanol was refluxed for 9 hours. The reaction mixture was concentrated under reduced pressure, and then water was added to the residue and the mixture was extracted with dichloromethane. The extract was dried over anhydrous sodium sulfate and then concentrated under reduced pressure. The residue was washed with iso-propylether to give 27.0 g of colorless crystals. Recrystallization from ethanol gave colorless prisms,
m.p. 193-194°C·

| Anal. C₁₄H₂₀ClN₃O₃ | | | |
|---|---|---|---|
| Calcd. | C: 53.59; | H: 6.42; | N: 13.39 |
| Found | C: 53.44; | H: 6.38; | N: 13.28 |

### Example 1: Ethyl endo-[3-(4-acetylamino-2-methoxybenzamido)-9-azabicyclo[3.3.1]non-9-yl]acetate

A mixture of 12.4 g of endo-4-acetylamino-N-(9-azabicyclo[3.3.1]non-3-yl)-2-methoxybenzamide, 4.6 ml of ethyl bromoacetate, 5.72 g of potassium carbonate, and 75 ml of N,N-dimethylformamide was heated for 2 hours at 60°C with stirring. The reaction mixture was poured into ice-water, and crystals precipitated were collected by filtration and washed with water to give 13.4 g of pale yellow crystals. Recrystallization from benzene gave colorless prisms, m.p. 85-88 °C·

| Anal. C₂₂ H₃₁N₃O₅ · 1/2H₂O | | | |
|---|---|---|---|
| Calcd. | C: 61.95; | H: 7.56; | N: 9.85 |
| Found | C: 61.85; | H: 7.55; | N: 9.86 |

In the same manner as Example 1, the compounds of Examples 2 to 12 were obtained.

### Example 2: Ethyl endo-4-[3-(4-acetylamino-2-methoxybenzamido)-9-azabicyclo[3.3.1]non-9-yl]butyrate

Appearance: colorless oil
IR Spectrum ν (liq) cm⁻¹ : 1732, 1698, 1638
NMR Spectrum δ (CDCl₃) ppm : 1.00-2.15(10H,m), 1.26(3H,t,J=7.0Hz), 2.21(3H,s), 2.37(2H,t,J=7.0Hz), 2.45-2.60(2H,m), 2.60-2.85(2H,m), 3.10-3.30 (2H,m), 3.96(3H,s), 4.13(2H,q,J=7.0Hz), 4.30-4.55(1H,m), 6.77(1H,dd,J=8.5,1.5Hz), 7.65-7.75(1H,m), 7.82(1H,s), 7.86(1H,d,J=1.5Hz), 8.09(1H,d,J=8.5Hz)

| High Resolution Mass Spectrum : C₂₄ H₃₅ N₃ O₅ | | |
|---|---|---|
| Calcd. | m/z : | 445.2577 |
| Found | m/z : | 445.2586 |

### Example 3: Methyl endo-6-[3-(4-acetylamino-2-methoxybenzamido)-9-azabicyclo[3.3.1]non-9-yl]hexanoate

Appearance: colorless oil
IR Spectrum ν (liq ) cm⁻¹ 1738, 1698, 1638
NMR Spectrum δ (CDCl₃) ppm: 1.00-2.00(14H,m), 2.21(3H,s), 2.33(2H,t,J=7.5Hz), 2.40-2.65(2H,m), 2.65-2.85(2H,m), 3.15-3.35(2H,m), 3.67(3H,s), 3.97(3H,s), 4.40-4.60(1H,m), 6.78(1H,d,J=8.0Hz), 7.65-7.70(1H,m), 7.70-7.80(1H,m), 7.85(1H,s), 8.10(1H,d,J=8.0Hz)

| High Resolution Mass Spectrum : C₂₅ H₃₇ N₃ O₅ | | |
|---|---|---|
| Calcd. | m/z : | 459.2733 |
| Found | m/z : | 459.2744 |

### Example 4: Methyl endo-8-[3-(4-acetylamino-2-methoxybenzamido)-9-azabicyclo[3.3.1]non-9-yl]octanoate

Appearance: colorless needles (EtOH)
m.p. 159-160°C
IR Spectrum ν (KBr) cm ⁻¹ : 1740, 1696, 1636
NMR Spectrum δ (CDCl₃) ppm : 1.00-2.15(18H,m), 2.21(3H,s), 2.30(2H,t,J=7.5Hz), 2.40-2.60(2H,m), 2.60-2.70(2H,m), 3.10-3.30(2H,m), 3.67(3H,s), 3.95(3H,s), 4.30-4.60(1H,m), 6.80(1H,dd,J=8.5,2.0Hz), 7.65-7.75(1H,m), 7.87(1H,m), 8.07(1H,d,J=8.5Hz), 8.26(1H,d,J=2.0Hz)

| High Resolution Mass Spectrum : C₂₇ H₄₁ N₃ O₅ | | |
|---|---|---|
| Calcd. | m/z : | 487.3046 |
| Found | m/z : | 487.3041 |

### Example 5: Ethyl endo-3-[3-(4-acetylamino-2-methoxybenzamido)-9-azabicyclo[3.3.1]non-9-yl]propionate

Appearance: colorless oil
IR Spectrum ν (KBr) cm ⁻¹ : 1732, 1698, 1638
NMR Spectrum δ (CDCl₃) ppm : 1.00-2.06(8H,m), 1.27(3H,t,J=7.0Hz), 2.21(3H,s), 2.32-2.54(2H,m), 2.40(2H,t,J=7.0Hz), 2.95(2H,t,J=7.0Hz), 3.06-3.20(2H,m), 3.95(3H,s), 4.15(2H,q,J=7.0Hz), 4.28-4.46(1H,m), 6.77(1H,dd,J=8.5,2.0Hz), 7.58-7.72(1H,m), 7.87(1H,br-s), 8.06(1H,d,J=8.5Hz), 8.19(1H,s)

| High Resolution Mass Spectrum : C₂₃ H₃₃ N₃ O₅ | | |
|---|---|---|
| Calcd. | m/z : | 431.2420 |
| Found | m/z : | 431.2420 |

### Example 6: Ethyl endo-5-[3-(4-acetylamino-2-methoxybenzamido)-9-azabicyclo[3.3.1]non-9-yl]valerate

Appearance: colorless oil
IR Spectrum ν (KBr) cm ⁻¹ : 1734, 1696, 1636
NMR Spectrum δ (CDCl₃) ppm: 0.95-2.10(12H,m), 1.26(3H,t,J=7.0Hz), 2.21(3H,s), 2.31(2H,t,J=7.0Hz), 2.37-2.55(2H,m), 2.64(2H,t,J=7.0Hz), 3.03-3.20(2H,m), 3.95(3H,s), 4.13(2H,q,J=7.0Hz), 4.33-4.53(1H,m), 6.77(1H,dd,J=8.5,2.0Hz),, 7.58-7.72(1H,m), 7.87 (1H,d,J=2.0Hz), 8.07(1H,d,J=8.5Hz), 8.09(1H,s)

| High Resolution Mnass Spectrum : C₂₅ H₃₇ N₃ O₅ | | |
|---|---|---|
| Calcd. | m/z : | 459.2733 |
| Found | m/z : | 459.2724 |

### Example 7: Methyl endo-7-[3-(4-acetylamino-2-methoxybenzamido)-9-azabicyclo[3.3.1]non-9-yl]heptanoate

Appearance: colorless oil
IR Spectrum ν (KBr) cm ⁻¹ : 1732, 1696, 1644
NMR Spectrum δ (CDCl₃) ppm : 1.00-2,10(16H,m), 2.21(3H,s), 2.30(2H,t,J=7.5Hz), 2.37-2.55(2H,m), 2.61(2H,t,J=7.0Hz), 3.05-3.20(2H,m), 3.66(3H,s), 3.93(3H,s), 4.35-4.55(1H,m), 6 .84(1H,dd,J=8.5,1.5Hz), 7.64-7.78(1H,m), 7.87(1H,d,J=1.5Hz), 8.03(1H,d,J=8.5Hz), 8.85(1H,s)

| High Resolution Mass Spectrum : C₂₆ H₃₉ N₃ O₅ | | |
|---|---|---|
| Calcd. | m/z : | 473.2890 |
| Found | m/z : | 473.2893 |

### Example 8: Ethyl endo-[3-(4-acetylamino-2-ethoxybenzamido)-9-azabicyclo[3.3.1]non-9-yl]acetate

Appearance: colorless needles (benzene)
m.p. 77-81°C

| Anal. C₂₃ H₃₃ N₃ O₅ · 1/2H₂ O | | | |
|---|---|---|---|
| Calcd. | C: 62.71; | H: 7.78; | N: 9.54 |
| Found | C: 62.72; | H: 7.57; | N: 9.55 |

### Example 9: Ethyl exo-[3-(4-acetylamino-2-methoxybenzamido)-9-azabicyclo[3.3.1]non-9-yl]acetate

Appearance: colorless oil
IR Spectrum ν (KBr) cm ⁻¹ : 1746, 1686, 1636
NMR Spectrum δ (CDCl₃) ppm : 1.28(3H,t,J=7.0Hz), 1.40-2.13(10H,m), 2.20(3H,s), 3.00-3.15(2H,m), 3.54(2H,s), 3.94(3H,s), 4.19(2H,q,J=7.0Hz), 4.80-5.00(1H,m), 6.78(1H,dd,J=8.5,2.0Hz), 7.57-7.75(1H,m), 7.83(1H,d,J=2.0Hz), 7.87(1H,s), 8.09(1H,d,J=8.5Hz)

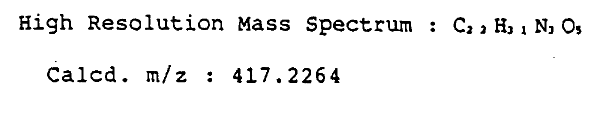

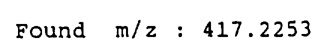

### Example 10: Ethyl exo-[3-(4-acetylamino-2-methoxybenzamido)-8-azabicyclo[3.2.1]oct-8-yl]acetate

Appearance: pale yellow oil
IR Spectrum ν (KBr) cm ⁻¹ : 1746, 1692, 1638
NMR Spectrum δ (CDCl₃) ppm: 1.28(3H,t,J=7.0Hz), 1.60-2.10(8H,m), 2.20(3H,s), 3.23(2H,s), 3.30-3.45(2H,m), 3.92(3H,s), 4.20(2H,q,J=7.0Hz), 4.28-4.48(1H,m), 6.77(1H,dd,J=8.5,2.0Hz), 7.60(1H,s), 7.60-7.80(1H,m), 7.80(1H,d,J=2.0Hz), 7.80(1H,s), 8.10(1H,d,J=8.5Hz)

| High Resolution Mass Spectrum : C₂₁ H₂₉ N₃ O₅ | | |
|---|---|---|
| Calcd. | m/z : | 403.2107 |
| Found | m/z : | 403.2111 |

### Example 11: Ethyl endo-3-[3-(4-acetylamino-5-chloro-2-methoxybenzamido)-8-azabicyclo[3.2.1]oct-8-yl]acetate

Appearance: colorless prisms (EtOH)
m.p. 144-145°C

| Anal. C₂₁ H₂₈ ClN₃ O₅ | | | |
|---|---|---|---|
| Calcd. | C: 57.60; | H: 6.44; | N: 9.60 |
| Found | C: 57.45; | H: 6.32; | N: 9.49 |

### Example 12: Ethyl endo-4-[3-(4-amino-5-chloro-2-methoxybenzamido)-8-azabicyclo[3.2.1]oct-8-yl]butyrate

Appearance: pale brown needles (AcOEt)
m.p. 154-156°C

| Anal. C₂₁ H₃₀ ClN₃ O₄ · 1/4H₂O | | | |
|---|---|---|---|
| Calcd. | C: 58.87; | H: 7.18; | N: 9.81 |
| Found | C: 58.80; | H: 6.97; | N: 9.71 |

### Example 13: Ethyl endo-[3-(4-acetylamino-5-chloro-2-methoxybenzamido)-9-azabicyclo[3.3.1]non-9-yl]acetate

To a suspension of 13.0 g of ethyl endo-[3-(4-acetylamino-2-methoxybenzamido)-9-azabicyclo[3.3.1]non-9-yl]acetate in 140 ml of dichloromethane, 3.4 ml of sulfuryl chloride was added dropwise at - 10°C. Siirring was continued at room temperature for 1.5 hours, and then the mixture was made basic by adding saturated aqueous sodium bicarbonate solution. The organic layer was separated and dried over anhydrous sodium sulfate. After being concentrated under reduced pressure, the residue was purified by column chromatography on silica gel (eluent: dichloromethane-methanol/50:1) to give 11.4 g of pale yellow amorphous solid.
IR Spectrum ν (KBr) cm ⁻¹ : 1750, 1646
NMR Spectrum δ (CDCl₃) ppm: 1.05-2.17(8H,m), 1.28(3H,t,J=7.3Hz), 2.28(3H,s), 2.42-2.64(2H,m), 3.12-3.34(2H,m), 3.49(2H,s), 3.98(3H,s), 4.17(2H,q,J=7.3Hz), 4.34-4.55(1H,m), 7.58-7.71(1H,m), 7.80(1H,s), 8.22(1H,s), 8.32(1H,s)

| High Resolution Mass Spectrum : C₂₂ H₃₀ ClN₃ O₅ | | | |
|---|---|---|---|
| Calcd. | m/z : | 451.1874, | 453.1844 |
| Found | m/z : | 451.1859, | 453.1853 |

In the same manner as Example 13, the compounds of Examples 14 to 22 were obtained.

### Example 14: Ethyl endo-4-[3-(4-acetylamino-5-chloro-2-methoxybenzamido)-9-azabicyclo[3.3.1]non-9-yl]butyrate

Appearance: colorless oil
IR Spectrum ν (liq) cm ⁻¹ : 1732, 1704, 1646
NMR Spectrum δ (CDCl₃) ppm: 1.00-2.10(10H,m), 1.27(3H,t,J=7.0Hz), 2.28(3H,s), 2.37(2H,t,J=7.5Hz), 2.45-2.60(2H,m), 2.65-2.80(2H,m), 3.05-3.20(2H,m), 3.98(3H,s), 4.14(2H,q,J=7.0Hz), 4.30-4.50(1H,m), 7.55-7.65(1H,m), 7.82(1H,s), 8.20(1H,s), 8.31(1H,s)

| High Resolution Mass Spectrum : C₂₄ H₃₄ ClN₃ O₅ | | | |
|---|---|---|---|
| Calcd. | m/z : | 479.2187, | 481.2157 |
| Found | m/z : | 479.2190, | 481.2173 |

### Example 15: Methyl endo-6-[3-(4-acetylamino-5-chloro-2-methoxybenzamido)-9-azabicyclo[3.3.1]non-9-yl]hexanoate fumarate

Appearance: colorless needles (EtOH)
m.p. 157-161°C

| Anal. C₂₅ H₃₆ ClN₃ O₅ · C₄ H₄ O₄ · 5/4H₂O | | | |
|---|---|---|---|
| Calcd. | C: 55.06; | H: 6.77; | N, 6.64 |
| Found | C: 54.95; | H: 6.94; | N, 6.72 |

### Example 16: Methyl endo-8-[3-(4-acetylamino-5-chloro-2-methoxybenzamido)-9-azabicyclo[3.3.1]non-9-yl]octanoate

Appearance: colorless needles (EtOH)
IR Spectrum ν (KBr) cm ⁻¹ : 1738, 1704, 1646
NMR Spectrum δ (CDCl₃ ) ppm: 1.00-2.10(18H,m), 2.28(3H,s), 2.31(2H,t,J=7.5Hz), 2.35-2.55(2H,m), 2.55-2.75(2H,m), 3.10-3.25(2H,m), 3.67(3H,s), 3.98(3H,s), 4.30-4.55(1H,m), 7.55-7.75(1H,m), 7.81(1H,s), 8.21(1H,s), 8.31(1H,s)

| High Resolution Mass Spectrum : C₂₇ H₄₀ ClN₃ O₅ | | | |
|---|---|---|---|
| Calcd. | m/z : | 521.2657, | 523.2627 |
| Found | m/z : | 521.2642, | 523.2617 |

### Example 17: Ethyl endo-3-[3-(4-acetylamino-5-chloro-2-methoxybenzamido)-9-azabicyclo[3.3.1]non-9-yl]propionate sesquifumarate

Appearance: colorless needles (EtOH)
m.p. 170-172°C

| Anal. C₂₃ H₃₂ ClN₃ O₅ · 3/2C₄ H₄ O₄ | | | |
|---|---|---|---|
| Calcd. | C: 54.42; | H: 5.98; | N: 6.56 |
| Found | C: 54.18; | H: 6.06; | N: 6.69 |

### Example 18: Ethyl endo-5-[3-(4-acetylamino-5-chloro-2-methoxybenzamido)-9-azabicyclo[3,3,l]non-9-yl]valerate fumarate

Appearance: colorless crystals (acetone)
m.p. 127-129°C

| Anal. C₂₅ H₃₆ ClN₃ O₅ · C₄ H₄ O₄ · 1/2H₂O | | | |
|---|---|---|---|
| Calcd. | C: 56.26; | H: 6.67; | N: 6.79 |
| Found | C: 56.34; | H: 6.72; | N: 6.65 |

### Example 19: Methyl endo-7-[3-(4-acetylamino-5-chloro-2-methoxybenzamido)-9-azabicyclo[3.3.1]non-9-yl]heptanoate fumarate

Appearance: colorless needles (MeOH-Et₂O)
m.p. 124.5-126°C

| Anal. C₂₆ H₃₈ ClN₃ O₅ · C₄ H₄ O₄ · 3/4H₂O | | | |
|---|---|---|---|
| Calcd. | C: 56.51; | H: 6.88; | N: 6.59 |
| Found | C: 56.51; | H: 6.82; | N: 6.59 |

### Example 20: Ethyl endo-[3-(4-acetylamino-5-chloro-2-ethoxybenzamido)-9-azabicyclo[3.3.1]non-9-yl]acetate

Appearance: slightly yellow syrup
IR Spectrum ν (liq) cm ⁻¹ : 1748, 1704, 1646
NMR Spectrum δ (CDCl₃) ppm: 1.05-1.64(5H,m), 1.75-2.10(3H,m), 2.45-2.65(2H,m), 1.28(3H,t,J=7.0Hz), 1.52(3H,t,J=7.0Hz), 2.27(3H,s), 3.16-3.31(2H,m), 3.49(2H,s), 4.18(2H,q,J=7.0Hz), 4.22(2H,q,J=7.0Hz), 4.37-4.55(1H,m), 7.78(1H,br-s), 7.80-7.94(1H,m), 8.21(1H,s), 8.27(1H,s)

| High Resolution Mass Spectrum : C₂₃ H₃₂ ClN₃ O₅ | | | |
|---|---|---|---|
| Calcd. | m/z : | 465.2031, | 467.2001 |
| Found | m/z : | 465.2033, | 467.2003 |

### Example 21: Ethyl exo-[3-(4-acetylamino-5-chloro-2-methoxybenzamido)-9-azabicyclo[3.3.1]non-9-yl]acetate 1/2 fumarate

Appearance: colorless needles (MeOH)
IR Spectrum ν (KBr) cm ⁻¹ : 1738, 1698, 1638
NMR Spectrum δ (DMSO-d₆) ppm: 1.19(3H,t,J=7.0Hz), 1.43-2.05(10H,m), 2.14(3H,s), 2.90-3.00(2H,m), 3.48(2H,s), 3.85(3H,s), 4.08(2H,q,J=7.0Hz), 4.55-4.73(1H,m), 6.61(1H,s), 7.69(1H,s), 7.73(1H,s), 7.70-7.90(1H,m), 9.42 (1H,s)

| High Resolution Mass Spectrum : C₂₂ H₃₀ ClN₃ O₅ | | | |
|---|---|---|---|
| Calcd. | m/z : | 451.1874, | 453.1844 |
| Found | m/z : | 451.1876, | 453.1840 |

### Example 22: Ethyl exo-[3-(4-acetylamino-5-chloro-2-methoxybenzamido)-8-azabicyclo[3.2.1]oct-8-yl]acetate maleate

Appearance: colorless crystals (EtOH)
m.p. 174-175°C

| Anal. C₂₁ H₂₈ ClN₃ O₅ · C₄ H₄ O₄ | | | |
|---|---|---|---|
| Calcd. | C: 54.20; | H: 5.82; | N: 7.58 |
| Found | C: 54.03; | H: 5.85; | N: 7.56 |

### Example 23: Ethyl endo-[3-(4-amino-5-chloro-2-methoxybenzamido)-9-azabicyclo[3.3.1]non-9-yl]acetate

To a solution of 11.0 g of ethyl endo-[3-(4-acetylamino-5-chloro-2-methoxybenzamido)-9-azabicyclo[3.3.1]non-9-yl]acetate in 22 ml of ethanol, 66 ml of 20% hydrogen chloride/ethanol solution was added and the mixture was refluxed for 1 hour. The reaction mixture was concentrated under reduced pressure, and then the residuce was dissolved in water and pH was adjusted to 10 with potassium carbonate. Crystals precipitated were collected by filtration and washed with water and then with isopropyl ether to give 8.88 g of pale brown crystals. Recrystallization from ethanol gave colorless crystals,
m.p. 163.5-164.5 °C.

| Anal. C₂₀ H₂₈ ClN₃ O₄ | | | |
|---|---|---|---|
| Calcd. | C: 58.60; | H: 6.88; | N: 10.25 |
| Found | C: 58.39; | H: 6.84; | N: 10.26 |

In the same manner as Example 23, the compounds of Examples 24 to 33 were obtained.

### Example 24: Ethyl endo-4-[3-(4-amino-5-chloro-2-methoxybenzamido)-9-azabicyclo[3.3.1]non-9-yl]butyrate

Appearance: pale orange prisms (EtOH)
m.p. 136.5-138°C

| Anal. C₂₂ H₃₂ ClN₃ O₄ | | | |
|---|---|---|---|
| Calcd. | C: 60.33; | H: 7.36; | N: 9.59 |
| Found | C: 60.27; | H: 7.41; | N: 9.52 |

### Example 25: Ethyl endo-6-[3-(4-amino-5-chloro-2-methoxybenzamido)-9-azabicyclo[3.3.1]non-9-yl]hexanoate

Appearance: colorless prisms (EtOH)
m.p. 122-123.5°C

| Anal. C₂₄ H₃₆ ClN₃ O₄ | | | |
|---|---|---|---|
| Calcd. | C: 61.86; | H: 7.79; | N: 9.02 |
| Found | C: 61.68; | H: 7.80; | N: 9.01 |

### Example 26: Ethyl endo-8-[3-(4-amino-5-chloro-2-methoxybenzamido)-9-azabicyclo[3.3.1]non-9-yl]octanoate

Appearance: colorless needles (EtOH)
m.p. 108-109°C

| Anal. C₂₆ H₄₀ ClN₃ O₄ | | | |
|---|---|---|---|
| Calcd. | C: 63.21; | H: 8.16; | N: 8.50 |
| Found | C: 62.93; | H: 8.14; | N: 8.46 |

### Example 27: Ethyl endo-3-[3-(4-amino-5-chloro-2-methoxybenzamido)-9-azabicyclo[3.3.1]non-9-yl]propionate

Appearance: pale pink needles (AcOEt)
m.p. 110-112°C

| Anal. C₂₁ H₃₀ ClN₃ O₄ | | | |
|---|---|---|---|
| Calcd. | C: 59.50; | H: 7.13; | N: 9.91 |
| Found | C: 59.31; | H: 7.12; | N: 9.89 |

### Example 28: Ethyl endo-5-[3-(4-amino-5-chloro-2-methoxybenzamido)-9-azabicyclo[3.3.1]non-9-yl]valerate

Appearance: colorless crystals (EtOH)
m.p. 126-128°C

| Anal. C₂₃ H₃₄ ClN₃ O₄ | | | |
|---|---|---|---|
| Calcd. | C: 61.12; | H: 7.58; | N: 9.30 |
| Found | C: 60.85; | H: 7.49; | N: 9.26 |

### Example 29: Methyl endo-7-[3-(4-amino-5-chloro-2-methoxybenzamido)-9-azabicyclo[3.3.1]non-9-yl]heptanoate

Appearance: colorless prisms (MeOH-Et₂O)
m.p. 144.5-145.5°C

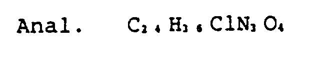

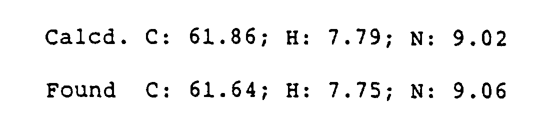

### Example 30: Ethyl endo-[3-(4-amino-5-chloro-2-ethoxybenzamido)-9-azabicyclo[3.3.1]non-9-yl]acetate

Appearance: colorless needles (EtOH)
m.p. 215-217°C

| Anal. C₂₁ H₃₀ ClN₃ O₄ | | | |
|---|---|---|---|
| Calcd. | C: 59.50; | H: 7.13; | N: 9.91 |
| Found | C: 59.26; | H, 7.04; | N: 9.87 |

### Example 31: Ethyl exo-[3-(4-amino-5-chloro-2-methoxybenzamido)-9-azabicyclo[3.3.1]non-9-yl]acetate

Appearance: pale pink crystals (iso-PrOH-Et₂O)
m.p. 161-164°C

| Anal. C₂₀ H₂₈ ClN₃ O₄ · 3/4H₂O | | | |
|---|---|---|---|
| Calcd. | C: 56.73; | H: 7.02; | N: 9.92 |
| Found | C: 56.73; | H: 6.63; | N: 9.96 |

### Example 32: Ethyl endo-[3-(4-amino-5-chloro-2-methoxybenzamido)-8-azabicyclo[3.2.1]oct-8-yl]acetate

Appearance: colorless plates (EtOH)
m.p. 187-188°C

| Anal. C₁₉ H₂₆ ClN₃ O₄ | | | |
|---|---|---|---|
| Calcd. | C: 57.65; | H: 6.62; | N: 10.61 |
| Found | C: 57.57; | H: 6.52; | N: 10.42 |

### Example 33: Ethyl exo-[3-(4-amino-5-chloro-2-methoxybenzamido)-8-azabicyclo[3.2.1]oct-8-yl]acetate

Appearance: pale orange prisms (EtOH-Et₂O)
m.p. 188.5-190°C

| Anal. C₁₉ H₂₆ ClN₃ O₄ | | | |
|---|---|---|---|
| Calcd. | C: 57.65; | H: 6.62; | N: 10.61 |
| Found | C: 57.60; | H: 6.57; | N: 10.49 |

### Example 34: endo-[3-(4-Amino-5-chloro-2-methoxybenzamido)-9-azabicyclo[3.3.1]non-9-yl]acetic acid hydrochloride

To a suspension of 8.00 g of ethyl endo-[3-(4-amino-5-chloro-2-methoxybenzamido)-9-azabicyclo[3.3.1]non-9-yl]acetate in 80 ml of methanol, 19.5 ml of 2N sodium hydroxide solution was added and the mixture was refluxed for 1 hour. After the reaction mixture was concentrated under reduced pressure, the residuce was dissolved in a small volume of water and pH was adjusted to 1 with 10% hydrochloric acid. Crystals precipitated were collected by filtration, washed with water, and then recrystallized from methanol-isopropyl ether to give 5.75 g of colorless powder, m.p. 189-194°C (decomp.).

| Anal. C₁₈ H₂₄ ClN₃ O₄ · HCl · H₂O | | | |
|---|---|---|---|
| Calcd. | C: 49.55; | H: 6.23; | N: 9.63 |
| Found | C: 49.38; | H: 5.99; | N: 9.49 |

In the same manner as Example 34, the compounds of Examples 35 to 45 were obtained.

### Example 35: endo-4-[3-(4-Amino-5-chloro-2-methoxybenzamido)-9-azabicyclo[3.3.1]non-9-yl]butyric acid hydrochloride

Appearance: colorless crystals (H₂O)
m.p. 166-167°C

| Anal. C₂₀ H₂₈ ClN₃ O₄ · HCl · 3/2H₂O | | | |
|---|---|---|---|
| Calcd. | C: 50.74; | H: 6.81; | N: 8.88 |
| Found | C: 50.68; | H: 6.66; | N: 8.91 |

### Example 36: endo-6-[3-(4-Amino-5-chloro-2-methoxybenzamido)-9-azabicyclo[3.3.1]non-9-yl]hexanoic acid hydrochloride

Appearance: colorless crystals (H₂O)
m.p. 237-239°C

| Anal. C₂₂ H₃₂ ClN₃ O₄ · HCl · 5/4H₂O | | | |
|---|---|---|---|
| Calcd. | C: 53.17; | H: 7.20; | N: 8.46 |
| Found | C: 53.32; | H: 7.01; | N: 8.48 |

### Example 37: endo-8-[3-(4-Amino-5-chloro-2-methoxybenzamido)-9-azabicyclo[3.3.1]non-9-yl]octanoic acid hydrochloride

Appearance: slightly orange crystals (H₂O)
m.p. 125-128°C

| Anal. C₂₄ H₃₆ ClN₃ O₄ · HCl · 5/4H₂O | | | |
|---|---|---|---|
| Calcd. | C: 54.91; | H: 7.58; | N: 8.00 |
| Found | C: 54.87; | H: 7.44; | N: 7.98 |

### Example 38: endo-3-[3-(4-Amino-5-chloro-2-methoxybenzamido)-9-azabicyclo[3.3.1]non-9-yl]propionic acid hydrochloride

Appearance: pale brown prisms (H₂O)
m.p. 157-160°C

| Anal. C₁₉ H₂₆ ClN₃ O₄ · HCl · 7/4H₂O | | | |
|---|---|---|---|
| Calcd. | C: 49.20; | H: 6.63; | N: 9.06 |
| Found | C: 49.27; | H: 6.41; | N: 9.07 |

### Example 39: endo-5-[3-(4-Amino-5-chloro-2-methoxybenzamido)-9-azabicyclo[3.3.1]non-9-yl]valeric acid hydrochloride

Appearance: pale pink crystals (H₂O)
m.p. 152-153.5°C

| Anal. C₂₁ H₃₀ ClN₃ O₄ · HCl · 7/4H₂O | | | |
|---|---|---|---|
| Calcd. | C: 51.27; | H: 7.06; | N: 8.54 |
| Found | C: 51.30; | H: 6.89; | N: 8.53 |

### Example 40: endo-7-[3-(4-Amino-5-chloro-2-methoxybenzamido)-9-azabicyclo[3.3.1]non-9-yl]heptanoic acid hydrochloride

Appearance: colorless needles (H₂O)
m.p. 130-133°C

| Anal. C₂₃ H₃₄ ClN₃ O₄ · HCl · 2H₂O | | | |
|---|---|---|---|
| Calcd. | C: 52.67; | H: 7.49; | N: 8.01 |
| Found | C: 52.61; | H: 7.21; | N: 8.14 |

### Example 41: endo-[3-(4-Amino-5-chloro-2-ethoxybenzamido)-9-azabicyclo[3.3.1]non-9-yl]acetic acid hydrochloride

Appearance: slightly yellow needles (H₂O)
m.p. 216-220°C (decomp.)

| Anal. C₁₉ H₂₆ ClN₃ O₄ · HCl · 5/2H₂O | | | |
|---|---|---|---|
| Calcd. | C: 47.80; | H: 6.76; | N: 8.80 |
| Found | C: 48.05; | H: 6.41; | N: 8.93 |

### Example 42: exo-[3-(4-Amino-5-chloro-2-methoxybenzamido)-9-azabicyclo[3.3.1] non-9-yl]acetic acid

Appearance: pale orange crystals (MeOH)
m.p. 250-252°C (decomp.)

| Anal. C₁₈ H₂₄ Cl N₃ O₄ · 3/4H₂O | | | |
|---|---|---|---|
| Calcd. | C: 54.68; | H: 6.50; | N: 10.63 |
| Found | C: 54.68; | H: 6.25; | N: 10.94 |

### Example 43: endo-[3-(4-Amino-5-chloro-2-methoxybenzamido)-8-azabicyclo[3.2.1]oct-8-yl]acetic acid hydrochloride

Appearance: slightly yellow needles (H₂O)
m.p. 254-256°C (decomp.)

| Anal. C₁₇ H₂₂ ClN₃ O₄ · HCl · 3/2H₂O | | | |
|---|---|---|---|
| Calcd. | C: 47.34; | H: 6.08; | N: 9.74 |
| Found | C: 47.36; | H: 5.96; | N: 9.79 |

### Example 44: endo-4-[3-(4-Amino-5-chloro-2-methoxybenzamido)-8-azabicyclo[3.2.1]oct-8-yl]butyric acid hydrochloride

Appearance: colorless crystals (H₂O)
m.p. 242-243°C

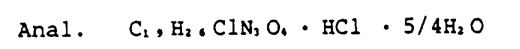

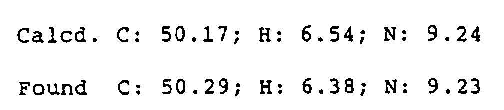

### Example 45: exo-[3-(4-Amino-5-chloro-2-methoxybenzamido)-8-azabicyclo[3.2.1]oct-8-yl]butyric acid

Appearance: pale orange crystals (MeOH-Et₂O)
m.p. 273-276°C (decomp.)

| Anal. C₁₇ H₂₂ ClN₃ O₄ | | | |
|---|---|---|---|
| Calcd. | C: 55.51; | H: 6.03; | N: 11.42 |
| Found | C: 55.20; | H: 6.05; | N: 11.20 |

### Example 46: Ethyl [4-(4-amino-5-chloro-2-methoxybenzamido)piperidino]acetate hydrochloride

A mixture of 3.20 g of 4-amino-5-chloro-2-methoxy-N-(4-piperidinyl)benzamide hydrochloride, 1.22 ml of ethyl bromoacetate, 3.04 g of porassium carbonate, and 32 ml of N,N-dimethylformamide was stirred for 2.5 hours with heating at 60 °C. The reaction mixture was concentrated under reduced pressure, and then the residue was added with water and extracted with dichloromethane. The extract was washed with saturated aqueous sodium chloride solution, dried over anhydrous sodium sulfate, and then concentrated under reduced pressure to give brown oil. The result was converted to the hydrochloride by an ordinary method to afford 3.31 g of pale yellow crystals. Recrystallization from ethanol gave colorless plates, m.p. 196.5-198.5 °C.

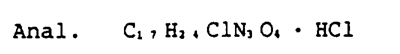

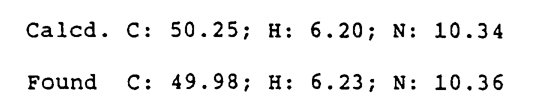

In the same manner as Exmple 46, the compounds of Examples 47-57 were obtained.

### Example 47: Ethyl 4-[4-(4-amino-5-chloro-2-methoxybenzamido)piperidino]butyrate hydrochloride

Appearance: slightly brown columns (EtOH)
m.p. 188.5-191.5°C

| Anal. C₁₉ H₂₈ ClN₃ O₄ · HCl | | | |
|---|---|---|---|
| Calcd. | C: 52.54; | H: 6.73; | N: 9.67 |
| Found | C: 52.18; | H: 6.66; | N: 9.75 |

### Example 48: Methyl 6-[4-(4-amino-5-chloro-2-methoxybenzamido)piperidino]hexanoate hydrochloride

Appearance: slightly brown needles (MeOH)
m.p. 208.5-210.5°C

| Anal. C₂₀ H₃₀ ClN₃ O₄ · HCl · 5/4H₂O | | | |
|---|---|---|---|
| Calcd. | C: 51.01; | H: 7.17; | N: 8.92 |
| Found | C: 51.06; | H: 7.03; | N: 8.99 |

### Example 49: Ethyl 3-[4-(4-amino-5-chloro-2-methoxybenzamido)piperidino]propionate

Appearance: colorless needles (acetone-Et₂O)
m.p. 116-117.5°C

| Anal. C₁₈ H₂₆ ClN₃ O₄ | | | |
|---|---|---|---|
| Calcd. | C: 56.32; | H: 6.83; | N: 10.95 |
| Found | C: 56.28; | H: 6.74; | N: 10.87 |

### Example 50: Ethyl 5-[4-(4-amino-5-chloro-2-methoxybenzamido)piperidino]valerate hydrochloride

Appearance: colorless crystals (EtOH)
m.p. 202-203.5°C

| Anal. C₂₀ H₃₀ ClN₃ O₄ · HCl · 1/4H₂O | | | |
|---|---|---|---|
| Calcd. | C: 53.04; | H: 7.01; | N: 9.28 |
| Found | C: 52.99; | H: 6.95; | N: 9.21 |

### Example 51: Ethyl 2-[4-(4-amino-5-chloro-2-methoxybenzamido)piperidino]propionate fumarate

Appearance: slightly yellow crystals (EtOH)
m.p. 158-159°C

| Anal. C₁₈ H₂₆ ClN₃ O₄ · C₄ H₄ O₄ | | | |
|---|---|---|---|
| Calcd. | C: 52.85; | H: 6.05; | N: 8.40 |
| Found | C: 52.67; | H: 5.94; | N: 8.39 |

### Example 52: Ethyl cis-[4-(4-amino-5-chloro-2-methoxybenzamido)-3-methoxypiperidino]acetate hydrochloride

Appearance: colorless needles (MeOH)
m.p. 225-226°C

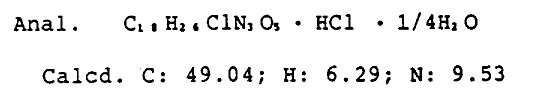

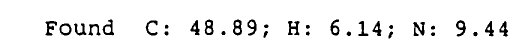

### Example 53: Ethyl cis-4-[4-(4-amino-5-chloro-2-methoxybenzamido)-3-methoxypiperidino]butyrate hydrochloride

Appearance: colorless crystals (EtOH)
m.p. 218-219.5°C

| Anal. C₂₀ H₃₀ ClN₃ O₅ · HCl | | | |
|---|---|---|---|
| Calcd. | C: 51.73; | H: 6.73; | N: 9.05 |
| Found | C: 51.45; | H: 6.64; | N: 8.98 |

### Example 54: Ethyl cis-6-[4-(4-amino-5-chloro-2-methoxybenzamido)-3-methoxypiperidino]hexanoate hydrochloride

Appearance: colorless crystals (EtOH)
m.p. 207-208.5°C

| Anal. C₂₂ H₃₄ ClN₃ O₅ · HCl · 1/4H₂O | | | |
|---|---|---|---|
| Calcd. | C: 53.17; | H: 7.20; | N: 8.46 |
| Found | C: 52.95; | H: 7.09; | N: 8.35 |

### Example 55: Ethyl [2-[(4-amino-5-chloro-2-methoxybenzamido)methyl]morpholino]acetate hydrochloride

Appearance: pale yellow crystals (H₂O)
m.p. 203-204°C

| Anal. C₁₇ H₂₄ ClN₃ O₅ · HCl | | | |
|---|---|---|---|
| Calcd. | C: 48.35; | H: 5.97; | N: 9.95 |
| Found | C: 48.12; | H: 6.03; | N: 9.75 |

### Example 56: Ethyl 4-[2-[(4-amino-5-chloro-2-methoxybenzamido)methyl]morpholino]butyrate hydrochloride

Appearance: pale yellow crystals (EtOH)
m.p. 196-197°C

| Anal. C₁₉ H₂₈ ClN₃ O₅ · HCl · 1/2H₂O | | | |
|---|---|---|---|
| Calcd. | C: 49.68; | H: 6.58; | N: 9.15 |
| Found | C: 49.59; | H: 6.65; | N: 9.10 |

### Example 57: Ethyl [3-[(4-amino-5-chloro-2-methoxybenzamido)methyl]pyrrolidin-1-yl]acetate

Appearance: pale yellow oil
IR Spectrum ν (liq.) cm⁻¹ : 1744, 1632
NMR Spectrum δ (CDCl₃) ppm: 1.27(3H,t,J=7.0Hz), 1.46-1.69(1H,m), 1.93-2.14(1H,m), 2.38-2.99(5H,m), 3.24-3.54(4H,m), 3.89(3H,s), 4.18(2H,q,J= 7.0Hz), 4.39(2H,br-s), 6.29(1H,s), 7.81(1H,br-s), 8.09(1H,s) High Resolution Mass Spectrum : C₁₇ H₂₄ ClN₃ O₄

| | | | |
|---|---|---|---|
| Calc. | m/z : | 369.1455, | 371.1426 |
| Found | m/z : | 369.1466, | 371.1439 |

### Example 58: [4-[(4-Amino-5-chloro-2-methoxybenzamido)piperidino]acetic acid hydrochloride

To a suspension of 2.23 g of ethyl [4-[(4-amino-5-chloro-2-methoxybenzamido)piperidino]acetate in 22 ml of methanol, 9,86 ml of 2N sodium hydroxide solution was added and the mixture was refluxed for 1 hour. After methanol was removed by distillation under reduced pressure, 10% hydrochloric acid was added to the residue and pH was adjusted to 2. After the mixture was cooled to 5 °C, crystals precipitated were collected by filtration and washed with water to give 1.47 g of pale brown crystals. Recrystallization from water gave pale yellow prisms, m.p. 248-251 °C (decomp.).

| Anal. C₁₅H₂₀ClN₃O₄ · HCl · 1/4H₂O | | | |
|---|---|---|---|
| Calcd. | C: 47.07; | H: 5.66; | N: 10.98 |
| Found | C: 47.34; | H: 5.58; | N: 11.08 |

In the same manner as Example 58, the compounds of Examples 59-68 were obtained.

### Example 59: 4-[4-(4-Amino-5-chloro-2-methoxybenzamido)piperidino]butyric acid hydrochloride

Appearance: pale yellow prisms (H₂O)
m.p. 228.5-231.5°C

| Anal. C₁₇ H₂₄ ClN₃ O₄ · HCl · H₂O | | | |
|---|---|---|---|
| Calcd. | C: 48.12; | H: 6.41; | N: 9.90 |
| Found | C: 47.97; | H: 6.34; | N: 10.12 |

### Example 60: 6-[4-(4-Amino-5-chloro-2-methoxybenzamido)piperidino]hexanoic acid hydrochloride

Appearance: slightly brown prisms (H₂O)
m.p. 223-225°C

| Anal. C₁₉ H₂₈ ClN₃ O₄ · HCl · H₂O | | | |
|---|---|---|---|
| Calcd. | C: 50.45; | H: 6.91; | N: 9.29 |
| Found | C: 50.33; | H: 6.84; | N: 9.25 |

### Example 61: 3-[4-(4-Amino-5-chloro-2-methoxybenzamido)piperidino]propionic acid hydrochloride

Appearance: colorless needles (H₂O)
m.p. 218-219.5°C

| Anal. C₁₆ H₂₂ ClN₃ O₄ · HCl | | | |
|---|---|---|---|
| Calcd. | C: 48.99; | H: 5.91; | N: 10.71 |
| Found | C: 48.80; | H: 5.84; | N: 10.68 |

### Example 62: 5-[4-(4-Amino-5-chloro-2-methoxybenzamido)piperidino]valeric acid hydrochloride

Appearance: colorless crystals (H₂O)
m.p. 226-227.5°C

| Anal. C₁₈ H₂₆ ClN₃ O₄ · HCl · H₂O | | | |
|---|---|---|---|
| Calcd. | C: 49.32; | H: 6.67; | N: 9.59 |
| Found | C: 49.18; | H: 6.45; | N: 9.54 |

### Example 63: 2-[4-(4-Amino-5-chloro-2-methoxybenzamido)piperidino]propionic acid

Appearance: pale yellow crystals (H₂O)
m.p. 246-247°C

| Anal. C₁₆ H₂₂ ClN₃ O₄ · 5/4H₂O | | | |
|---|---|---|---|
| Calcd. | C: 50.79; | H: 6.53; | N: 11.11 |
| Found | C: 50.59; | H: 6.25; | N: 11.06 |

### Example 64: cis-[4-(4-Amino-5-chloro-2-methoxybenzamido)-3-methoxypiperidino]acetic acid

Appearance: colorless needles (H₂O)
m.p. 230-233°C (decomp.)

| Anal. C₁₆ H₂₂ ClN₃ O₅ · 5/2H₂O | | | |
|---|---|---|---|
| Calcd. | C: 46.10; | H: 6.53; | N: 10.08 |
| Found | C: 46.18; | H: 6.57; | N: 10.00 |

### Example 65: cis-4-[4-(4-Amino-5-chloro-2-methoxybenzamido)-3-methoxypiperidino]butyric acid hydrochloride

Appearance: colorless needles (H₂O)
m.p. 232-234.5°C (decomp.)

| Anal. C₁₈ H₂₆ ClN₃O₅ · HCl · 1/4H₂O | | | |
|---|---|---|---|
| Calcd. | C: 49.04; | H: 6.29; | N: 9.53 |
| Found | C: 49.03; | H: 6.19; | N: 9.77 |

### Example 66: cis-6-[4-(4-Amino-5-chloro-2-methoxybenzamido)-3-methoxypiperidino]hexanoic acid hydrochloride

Appearance: colorless prisms (H₂O)
m.p. 232.5-235.5°C (decomp.)

| Anal. C₂₀ H₃₀ ClN₃ O₅ · HCl | | | |
|---|---|---|---|
| Calcd. | C: 51.73; | H: 6.73; | N: 9.05 |
| Found | C: 51.52; | H: 6.62; | N: 8.90 |

### Example 67: [2-[(4-Amino-5-chloro-2-methoxybenzamido)methyl]morpholino]acetic acid

Appearance: slightly yellow crystals (MeOH)
m.p. 208-210°C

| Anal. C₁₅ H₂₀ ClN₃ O₅ | | | |
|---|---|---|---|
| Calcd. | C: 50.35; | H: 5.63; | N: 11.74 |
| Found | C: 50.04; | H: 5.53; | N: 11.63 |

### Example 68: 4-[2-[(4-Amino-5-chloro-2-methoxybenzamido)methyl]morpholino]butyric acid

Appearance: colorless needles (EtOH)
m.p. 194-195°C

| Anal. C₁₇ H₂₄ ClN₃ O₅ | | | |
|---|---|---|---|
| Calcd. | C: 52.92; | H: 6.27; | N: 10.89 |
| Found | C: 52.60; | H: 6.09; | N: 10.83 |

### Formulation 1

| | |
|---|---|
| Compound of Example 23 | 5 mg |
| Lactose | suitable amount |
| Cornstarch | 15 mg |
| Magnesium Stearate | 1 mg |
| | 80 mg |

### Formulation 2

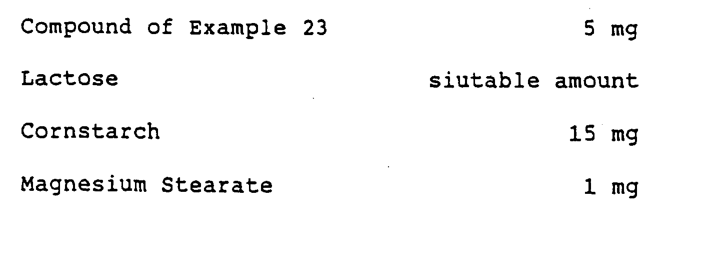

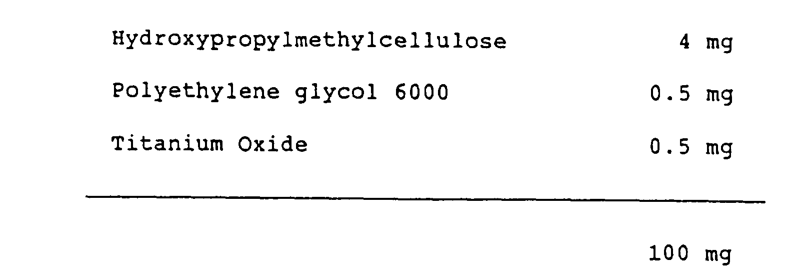

### Formulation 3

| | |
|---|---|
| Compound of Example 23 | 10 mg |
| Lactose | siutable amount |
| D-mannitol | 500 mg |
| Hydroxypropylcellulose | 20 mg |
| Talc | 2 mg |
| | 1,000 mg |

### Formulation 4

| | |
|---|---|
| Compound of Example 23 | 5 mg |
| Citric Acid | 0.5 mg |
| Glucose | 50 mg |
| Sodium Hydroxide | siutable amount |
| Distilled Water for Injection | siutable amount |
| | 1 ml |

### Formulation 5

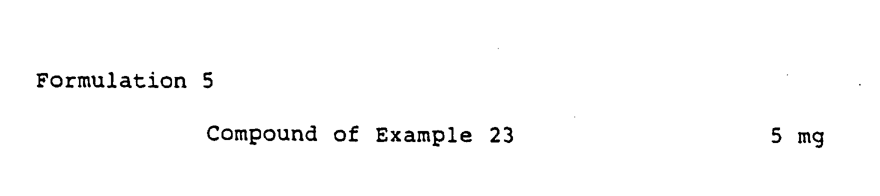

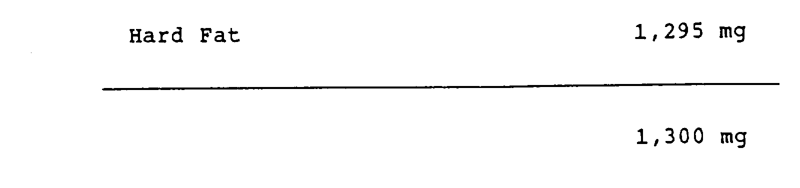

### Industrial Applicability

As explained above, the novel benzamide derivatives of the present invention represented by formula (I) have excellent gastrointestinal stimulating activity and antiemetic activity and thus are useful for the treatment of gastrointestinal diseases.

## Claims

1. A benzamide derivative represented by the following formula: 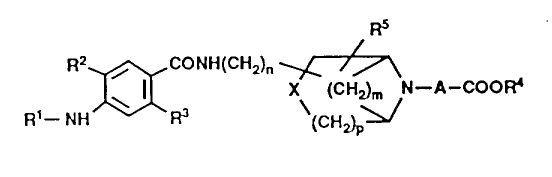 wherein, R¹ represents a hydrogen atom or a C₁-C₄ alkanoyl group; R² represents a hydrogen atom or a halogen atom; R³ represents a C₁-C₄ alkoxy group; R⁴ represents a hydrogen atom or a C₁-C₄ alkyl group; R⁵ represents a hydrogen atom, a C₁-C₄ alkyl group, or a C₁-C₄ alkoxy group; A represents C₁-C₇ alkylene group which may optionally be substituted with a C₁-C₄ alkyl group; X represents a methylene group, an oxygen atom, or a sulfur atom; m represents an integer of from 0 to 3; n represents an integer of from 0 to 3; and p represents an integer of from 0 to 2, and a pharmacologically acceptable salt thereof.

2. A compound according to claim 1, which is Ethyl endo-[3-(4-amino-5-chloro-2-methoxybenzamido)-9-azabicyclo[3.3.1]non-9-yl]acetate and a pharmacologically acceptable salt thereof.

3. A compound according to claim 1, which is Endo-[3-(4-amino-5-chloro-2-methoxybenzamido)-9-azabicyclo[3.3.1]non-9-yl]acetic acid and a pharmacologically acceptable salt thereof.

4. A compound according to claim 1, which is Ethyl endo-4-[3-(4-amino-5-chloro-2-methoxybenzamido)-9-azabicyclo[3.3.1]non-9-yl]butyrate and a pharmacologically acceptable salt thereof.

5. A compound according to claim 1, which is Endo-4-[3-(4-amino-5-chloro-2-methoxybenzamido)-9-azabicyclo[3.3.1]non-9-yl]butyric acid and a pharmacologically acceptable salt thereof.

6. A compound according to claim 1, which is Ethyl endo-[3-(4-amino-5-chloro-2-methoxybenzamido)-8-azabicyclo[3.2.1]oct-8-yl]acetate and a pharmacologically acceptable salt thereof.

7. A compound according to claim 1, which is Endo-[3-(4-amino-5-chloro-2-methoxybenzamido)-8-azabicyclo[3.2.1]oct-8-yl]acetic acid and a pharmacologically acceptable salt thereof.

8. A compound according to claim 1, which is Ethyl endo-4-[3-(4-amino-5-chloro-2-methoxybenzamido)-8-azabicyclo[3.2.1]oct-8-yl]butyrate and a pharmacologically acceptable salt thereof.

9. A compound according to claim 1, which is Endo -4-[3-(4-amino-5-chloro-2-methoxybenzamido)-8-azabicyclo[3.2.1]oct-8-yl]butyric acid and a pharmacologically acceptable salt thereof.

10. A compound according to claim 1, which is Ethyl [4-(4-amino-5-chloro-2-methoxybenzamido)piperidino]acetate and a pharmacologically acceptable salt thereof.

11. A compound according to claim 1, which is [4-(4-amino-5-chloro-2-methoxybenzamido)piperidino]acetic acid and a pharmacologically acceptable salt thereof.

12. A process for preparing a benzamide derivative represented by the following formula: 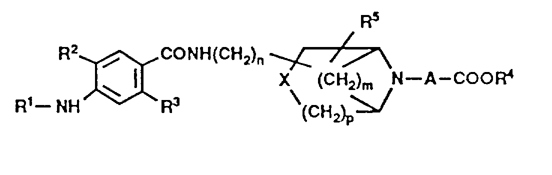 wherein R¹ represents a hydrogen atom or a C₁-C₄ alkanoyl group; R² represents a hydrogen atom or a halogen atom; R³ represents a C₁-C₄ alkoxy group; R⁴ represents a hydrogen atom or a C₁-C₄ alkyl group; R⁵ represents a hydrogen atom, a C₁-C₄ alkyl group, or a C₁-C₄ alkoxy group; A represents C₁-C₇ alkylene group which may optionally be substituted with a C₁-C₄ alkyl group; X represents a methylene group, an oxygen atom, or a sulfur atom; m represents an integer of from 0 to 3; n represents an integer of from 0 to 3; and p represents an integer of from 0 to 2 and a pharmacologically acceptable salt thereof, which comprises the steps of:
(a) reacting a compound represented by the following formula: 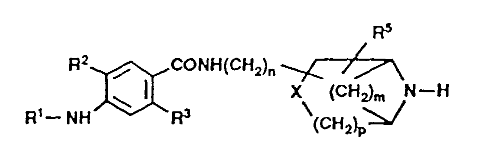 wherein R¹ represents a hydrogen atom or a C₁-C₄ alkanoyl group; R² represents a hydrogen atom or a halogen atom; R³ represents a C₁-C₄ alkoxy group; R⁵ represents a hydrogen atom, a C₁-C₄ alkyl group, or a C₁-C₄ alkoxy group; X represents a methylene group, an oxygen atom, or a sulfur atom; m represents an integer of from 0 to 3; n represents an integer of from 0 to 3; and p represents an integer of from 0 to 2,
with a compound represented by the following formula:
Y-A-CO₂ R⁶ or CH=CH₂ CO₂ R⁶ (III)
wherein R⁶ represents a C₁-C₄ alkyl, A represents C₁-C₇ alkylene group which may optionally be substituted with a C₁-C₄ alkyl group, and Y represents a halogen atom, and
(b) halogenating or hydrolyzing the product obtained by the above step (a), if desired.

13. A pharmaceutical composition comprising a benzamide derivative represented by the following formula: 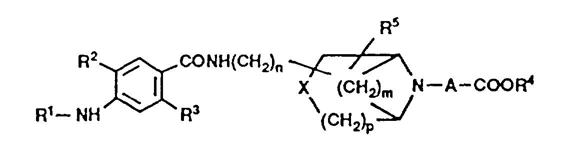 wherein R¹ represents a hydrogen atom or a C₁-C₄ alkanoyl group; R² represents a hydrogen atom or a halogen atom; R³ represents a C₁-C₄ alkoxy group; R⁴ represents a hydrogen atom or a C₁-C₄ alkyl group; R⁵ represents a hydrogen atom, a C₁-C₄ alkyl group, or a C₁-C₄ alkoxy group; A represents C₁-C₇ alkylene group which may optionally be substituted with a C₁-C₄ alkyl group; X represents a methylene group, an oxygen atom, or a sulfur atom; m represents an integer of from 0 to 3; n represents an integer of from 0 to 3; and p represents an integer of from 0 to 2 or a pharmacologically acceptable salt thereof as an active ingredient.

14. The composition according to claim 13, which is useful for the treatment of gastrointestinal diseases.

## Patentansprüche

1. Benzamid-Derivat, dargestellt durch die folgende Formel 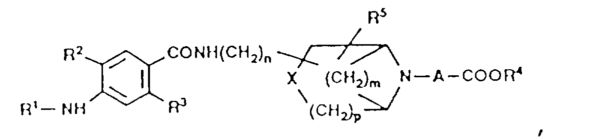 worin R¹ ein Wasserstoffatom oder eine C₁-C₄-Alkanoyl-Gruppe darstellt, R² ein Wasserstoffatom oder ein Halogenatom darstellt; R³ eine C₁-C₄-Alkoxy-Gruppe darstellt, R⁴ ein Wasserstoffatom oder eine C₁-C₄-Alkyl-Gruppe darstellt, R⁵ ein Wasserstoffatom, eine C₁-C₄-Alkyl-Gruppe oder eine C₁-C₄-Alkoxy-Gruppe darstellt; A eine C₁-C₇-Alkylengruppe darstellt, die gegebenenfalls mit einer C₁-C₄-Alkyl-Gruppe substituiert sein kann; X eine Methylengruppe, ein Sauerstoffatom oder Schwefelatom darstellt; m eine ganze Zahl von 0 bis 3 darstellt; n eine ganze Zahl von 0 bis 3 darstellt; und p eine ganze Zahl von 0 bis 2 darstellt; und ein pharmakologisch annehmbares Salz desselben.

2. Verbindung gemäß Anspruch 1, die Ethyl-endo-[3-(4-amino-5-chlor-2-methoxybenzamido)-9-azabicyclo[3,3,1]non-9-yl]acetat ist, und ein pharmakologisch annehmbares Salz derselben.

3. Verbindung gemäß Anspruch 1, die Endo-[3-(4-amino-5-chlor-2-methoxybenzamido)-9-azabicyclo[3.3.1]non-9-yl]-essigsäure ist; und ein pharmakologisch annehmbares Salz derselben.

4. Verbindung gemäß Anspruch 1, die Ethyl-endo-4-[3-(4-amino-5-chlor-2-methoxybenzamido)-9-azabicyclo[3.3.1]non-9-yl]butyrat ist, und ein pharmakologisch annehmbares Salz derselben.

5. Verbindung gemäß Anspruch 1, die Endo-4-[3-(4-amino-5-chlor-2-methoxybenzamido)-9-azabicyclo[3.3.1]non-9-yl]buttersäure ist; und ein pharmakologisch annehmbares Salz derselben.

6. Verbindung gemäß Anspruch 1, die Ethyl-endo-[3-(4-amino-5-chlor-2-methoxybenzamido)-8-azabicyclo[3.2.1]oct-8-yl]acetat ist, und ein pharmakologisch annehmbares Salz derselben.

7. Verbindung gemäß Anspruch 1, die Endo-[3-(4-amino-5-chlor-2-methoxybenzamido)-8-azabicyclo[3.2.1]oct-8-yl]essigsäure ist; und ein pharmakologisch annehmbares Salz derselben.

8. Verbindung gemäß Anspruch 1, die Ethyl-endo-4-[3-(4-amino-5-chlor-2-methoxybenzamido)-8-azabicyclo[3.2.1]oct-8-yl]butyrat ist, und ein pharmakologisch annehmbares Salz derselben.

9. Verbindung gemäß Anspruch 1, die Endo-4-[3-(4-amino-5-chlor-2-methoxybenzamido)-8-azabicyclo[3.2.1]oct-8-yl]butteräure ist; und ein pharmakologisch annehmbares Salz derselben.

10. Verbindung gemäß Anspruch 1, die Ethyl[4-(4-amino-5-chlor-2-methoxybenzamido)-piperidino]acetat ist, und ein pharmakologisch annehmbares Salz derselben.

11. Verbindung gemäß Anspruch 1, die [4-(4-Amino-5-chlor-2-methoxybenzamido)piperidino]essigsäure ist, und ein pharmakologisch annehmbares Salz derselben.

12. Verfahren zur Herstellung eines Benzamid-Derivats, dargestellt durch die folgende Formel: 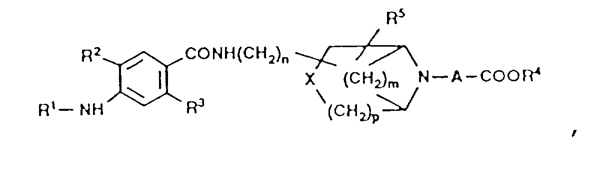 worin R¹ ein Wasserstoffatom oder eine C₁-C₄-Alkanoyl-Gruppe darstellt, R² ein Wasserstoffatom oder ein Halogenatom darstellt; R³ eine C₁-C₄-Alkoxy-Gruppe darstellt, R⁴ ein Wasserstoffatom oder eine C₁-C₄-Alkyl-Gruppe darstellt, R⁵ ein Wasserstoffatom, eine C₁-C₄-Alkyl-Gruppe oder eine C₁-C₄-Alkoxy-Gruppe darstellt; A eine C₁-C₇-Alkylengruppe darstellt, die gegebenenfalls mit einer C₁-C₄-Alkyl-Gruppe substituiert sein kann; X eine Methylengruppe, ein Sauerstoffatom oder Schwefelatom darstellt; m eine ganze Zahl von 0 bis 3 darstellt; n eine ganze Zahl von 0 bis 3 darstellt; und p eine ganze Zahl von 0 bis 2 darstellt; und ein pharmakologisch annehmbares Salz desselben, umfassend die Stufen:
(a) Umsetzung einer Verbindung, dargestellt durch die folgende Formel: 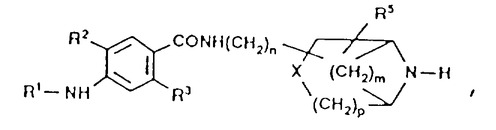 worin R¹ ein Wasserstoffatom oder eine C₁-C₄-Alkanoyl-Gruppe darstellt, R² ein Wasserstoffatom oder ein Halogenatom darstellt; R³ eine C₁-C₄-Alkoxy-Gruppe darstellt, R⁴ ein Wasserstoffatom oder eine C₁-C₄-Alkyl-Gruppe darstellt, R⁵ ein Wasserstoffatom, eine C₁-C₄-Alkyl-Gruppe oder eine C₁-C₄-Alkoxy-Gruppe darstellt; X eine Methylengruppe, ein Sauerstoffatom oder Schwefelatom darstellt; m eine ganze Zahl von 0 bis 3 darstellt; n eine ganze Zahl von 0 bis 3 darstellt, und p eine ganze Zahl von 0 bis 2 darstellt,
mit einer Verbindung, dargestellt durch die folgende Formel:
Y-A-CO₂R⁶ oder CH=CH₂CO₂R⁶ (III),
worin R⁶ eine C₁-C₄-Alkylgruppe darstellt, A eine C₁-C₇-Alkylengruppe darstellt, die gegebenenfalls mit einer C₁-C₄-Alkylgruppe substituiert sein kann, und Y ein Halogenatom darstellt, und
(b) Halogenieren oder Hydrolysieren des durch die obige Stufe (a) erhaltenen Produkts, falls erwünscht.

13. Pharmazeutische Zusammensetzung, umfassend ein Benzamid-Derivat, dargestellt durch die folgende Formel: 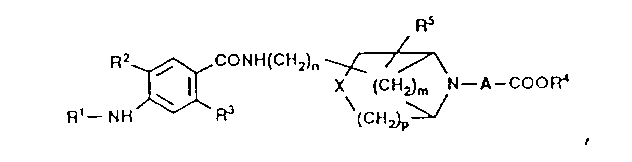 worin R¹ ein Wasserstoffatom oder eine C₁-C₄-Alkanoyl-Gruppe darstellt, R² ein Wasserstoffatom oder ein Halogenatom darstellt; R³ eine C₁-C₄-Alkoxy-Gruppe darstellt, R⁴ ein Wasserstoffatom oder eine C₁-C₄-Alkyl-Gruppe darstellt, R⁵ ein Wasserstoffatom, eine C₁-C₄-Alkyl-Gruppe oder eine C₁-C₄-Alkoxy-Gruppe darstellt; A eine C₁-C₇-Alkylengruppe darstellt, die gegebenenfalls mit einer C₁-C₄-Alkyl-Gruppe substituiert sein kann; X eine Methylengruppe, ein Sauerstoffatom oder Schwefelatom darstellt; m eine ganze Zahl von 0 bis 3 darstellt; n eine ganze Zahl von 0 bis 3 darstellt; und p eine ganze Zahl von 0 bis 2 darstellt; oder ein pharmakologisch annehmbares Salz derselben als einem aktiven Bestandteil.

14. Zusammensetzung gemäß Anspruch 13, welche zur Behandlung von Magenerkrankungen brauchbar ist.

## Revendications

1. Un dérivé de benzamide représenté par la formule suivante : 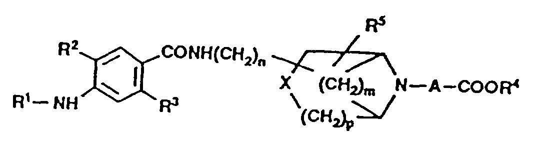 dans laquelle R¹ représente un atome d'hydrogène ou un groupe alkanoyle en C₁-C₄ ; R² repésente un atome d'hydrogène ou un atome d'un halogène ; R³ représente un groupe alkoxy en C₁-C₄ ; R⁴ représente un atome d'hydrogène ou un groupe alcoyle en C₁-C₄; R⁵ représente un atome d'hydrogène, un groupe alcoyle en C₁-C₄ ou un groupe alkoxy en C₁-C₄ ; A représente un groupe alkylène en C₁-C₇ qui peut optionnellement être substitué par un groupe alcoyle en C₁-C₄ ; X représente un groupe méthylène, un atome d'oxygène ou un atome de soufre ; m représente un entier de 0 à 3 ; n représente un entier de 0 à 3 ; et p représente un entier de 0 à 2, et un sel pharmacologiquement acceptable de celui-ci.

2. Un composé selon la revendication 1,
qui est l'endo-[3-(4-amino-5-chlore-2-méthoxybenzamido)-9-azabicyclo[3.3.l]non-9-yl]acétate d'éthyle et un sel pharmacologiquement acceptable de celui-ci.

3. Un composé selon la revendication 1,
qui est l'acide endo-[3-(4-amino-5-chloro-2-méthoxybenzamido)-9-azabicyclo[3.3.1]non-9-yl]acétique et un sel pharmacologiquement acceptable de celui-ci.

4. Un composé selon la revendication 1,
qui est l'endo-4-[3-(4-amino-5-chloro-2-méthoxybenzamido)-9-azabicyclo[3.3.1]non-9-yl]butyrate d'éthyle et un sel pharmacologiquement acceptable de celui-ci.

5. Un composé selon la revendication 1,
qui est l'acide endo-4-[3-(4-amino-5-chloro-2-méthoxybenzamido)-9-azabicyclo[3.3.1]non-9-yl]butyrique et un sel pharmacologiquement acceptable de celui-ci.

6. Un composé selon la revendication 1,
qui est l'endo-[3-(4-amino-5-chloro-2-méthoxybenzamido)-8-azabicyclo[3.2.1]oct-8-yl]acétate d'éthyle et un sel pharmacologiquement acceptable de celui-ci.

7. Un composé selon la revendication 1,
qui est l'acide endo-[3-(4-amino-5-chloro-2-méthoxybenzamido)-8-azabicyclo[3.2.1]oct-8-yl]acétique et un sel pharmacologiquement acceptable de celui-ci.

8. Un composé selon la revendication 1,
qui est l'endo-4-[3-(4-amino-5-chloro-2-méthoxybenzamido)-8-azabicyclo[3.2.1]oct-8-yl]butyrate d'éthyle et un sel pharmacologiquement acceptable de celui-ci.

9. Un composé selon la revendication 1,
qui est l'acide endo-4-[3-(4-amine-5-chloro-2-méthoxybenzamido)-8-azabicyclo[3.2.1]oct-8-yl]butyrique et un sel pharmacologiquement acceptable de celui-ci.

10. Un composé selon la revendication 1,
qui est le [4-(4-amino-5-chloro-2-méthoxybenzamido)pipéridino]acétate d'éthyle et un sel pharmacologiquement acceptable de celui-ci.

11. Un composé selon la revendication 1,
qui est l'acide [4-(4-amino-5-chloro-2-méthoxybenzamido)pipéridino]acétique et un sel pharmacologiquement acceptable de celui-ci.

12. Un procédé pour la préparation d'un dérivé de benzamide représenté par la formule suivante : 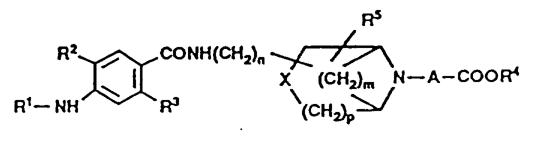 dans laquelle R¹ représente un atome d'hydrogène ou un groupe alkanoyle en C₁-C₄ ; R² repésente un atome d'hydrogène ou un atome d'un halogène ; R³ représente un groupe alkoxy en C₁-C₄ ; R⁴ représente un atome d'hydrogène ou un groupe alcoyle en C₁-C₄ ; R⁵ représente un atome d'hydrogène, un groupe alcoyle en C₁-C₄ ou un groupe alkoxy en C₁-C₄; A représente un groupe alkylène en C₁-C₇ qui peut optionnellement être substitué par un groupe alcoyle en C₁-C₄ ; X représente un groupe méthylène, un atome d'oxygène ou un atome de soufre ; m représente un entier de 0 à 3 ; n représente un entier de 0 à 3 ; et p représente un entier de 0 à 2 et un sel pharmacologiquement acceptable de celui-ci, qui comprend les étapes de :
(a) réaction d'un composé représenté par la formule suivante : 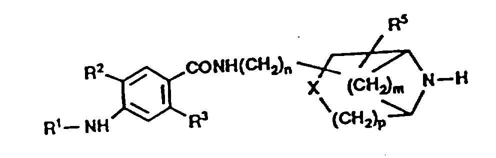 dans laquelle R¹ représente un atome d'hydrogène ou un groupe alkanoyle en C₁-C₄ ; R² repésente un atome d'hydrogène ou un atome d'un halogène R³ représente un groupe alkoxy en C₁-C₄ ; R⁵ représente un atome d'hydrogène, un groupe alcoyle en C₁-C₄ ou un groupe alkoxy en C₁-C₄; X représente un groupe méthylène, un atome d'oxygène ou un atome de soufre ; m représente un entier de 0 à 3 ; n représente un entier de 0 à 3 ; et p représente un entier de 0 à 2, avec un composé représenté par la formule suivante :
Y-A-CO₂R⁶ or CH=CH₂CO₂ R⁶ (III)
dans laquelle R⁶ représente un groupe alcoyle en C₁-C₄, A représente un groupe alkylène en C₁-C₇ qui peut optionnellement être substitué par un groupe alcoyle en C₁-C₄ et Y représente un atome d'halogène, et
(b) halogénation ou hydrolyse du produit obtenu par l'étape (a) ci-dessus, si désiré.

13. Une composition pharmaceutique comprenant un dérivé de benzamide représenté par la formule suivante : 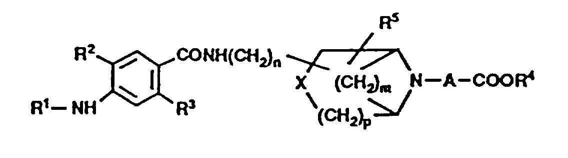 dans laquelle R¹ représente un atome d'hydrogène ou un groupe alkanoyle en C₁-C₄ ; R² repésente un atome d'hydrogène ou un atome d'un halogène ; R³ représente un groupe alkoxy en C₁-C₄ ; R⁴ représente un atome d'hydrogène ou un groupe alcoyle en C₁-C₄ ; R⁵ représente un atome d'hydrogène, un groupe alcoyle en C₁-C₄ ou un groupe alkoxy en C₁-C₄B A représente un groupe alkylène en C₁-C₇ qui peut optionnellement être substitué par un groupe alcoyle en C₁-C₄ ; X représente un groupe méthylène, un atome d'oxygène ou un atome de soufre ; m représente un entier de 0 à 3 ; n représente un entier de 0 à 3 ; et p représente un entier de 0 à 2 ou un sel pharmacologiquement acceptable de celui-ci, en tant qu'un ingrédient actif.

14. La composition selon la revendication 13,
qui est utile pour le traitement de maladies gastrointestinales.
